# EUROPEAN PATENT SPECIFICATION

(11) **EP 2 427 494 B1**
(45) Date of publication and mention of the grant of the patent: **18.10.2017**
(21) Application number: 10717658.8
(22) Date of filing: 07.05.2010
(51) Int. Cl.: C07K 16/22, A61P 43/00

(54) **METHODS OF MODULATING FIBROSIS USING BONE MORPHOGENETIC PROTEIN-9 (BMP-9) MODULATORS**
METHODEN ZUR MODULATION VON FIBROSE MITTELS BMP-9 MODULATOREN
PROCÉDÉS DE MODULATION DE LA FIBROSE UTILISANT DES MODULATEURS DE BMP-9

(30) Priority: 08.05.2009 US 215694 P
(43) Date of publication of application: 14.03.2012
(73) Proprietor: Novartis AG, 4056 Basel (CH)
(72) Inventor: BUCKLER, Alan, Cambridge, MA 02139 (US); CHEN, Chao-Min, Cambridge, MA 02139 (US); GUY, Chantale, Cambridge, MA 02139 (US); HEWETT, Jeffrey, Cambridge, MA 02139 (US)
(74) Representative: Kassow, Anders
(86) International application number: PCT/EP2010/056298
(87) International publication number: WO 2010/128158

(56) References cited:
- WO-A1-2008/151078
- ANDRIEUX ET AL: "Bone morphogenetic protein antagonist gene NOG is involved in myeloproliferative disease associated with myelofibrosis" CANCER GENETICS AND CYTOGENETICS, ELSEVIER SCIENCE PUBLISHING, NEW YORK, NY, US LNKD- DOI:10.1016/J.CANCERGENCYTO.2007.06.001, vol. 178, no. 1, 21 September 2007 (2007-09-21), pages 11-16, XP022263198 ISSN: 0165-4608
- ZEISBERG M ET AL: "The role of epithelial-to-mesenchymal transition in renal fibrosis" JOURNAL OF MOLECULAR MEDICINE, SPRINGER VERLAG, DE LNKD- DOI:10.1007/S00109-003-0517-9, vol. 82, no. 3, 1 March 2004 (2004-03-01), pages 175-181, XP003015976 ISSN: 0946-2716
- KLAHR SAULO: "The bone morphogenetic proteins (BMPs). Their role in renal fibrosis and renal function" JN. JOURNAL OF NEPHROLOGY, MILAN, IT, vol. 16, no. 2, 1 March 2003 (2003-03-01), pages 179-185, XP009128620 ISSN: 1121-8428
- TOBIN ET AL: "Bone morphogenetic proteins and growth differentiation factors as drug targets in cardiovascular and metabolic disease" DRUG DISCOVERY TODAY, ELSEVIER, RAHWAY, NJ, US LNKD- DOI:10.1016/J.DRUDIS.2006.03.016, vol. 11, no. 9-10, 1 May 2006 (2006-05-01) , pages 405-411, XP005402920 ISSN: 1359-6446
- RUIZ-ORTEGA ET AL: "TGF-beta signaling in vascular fibrosis" CARDIOVASCULAR RESEARCH, OXFORD UNIVERSITY PRESS, GB LNKD- DOI:10.1016/J.CARDIORES.2007.02.008, vol. 74, no. 2, 5 April 2007 (2007-04-05), pages 196-206, XP022033846 ISSN: 0008-6363
- LIU YOUHUA: "Renal fibrosis: New insights into the pathogenesis and therapeutics" KIDNEY INTERNATIONAL, vol. 69, no. 2, January 2006 (2006-01), pages 213-217, XP009137666 ISSN: 0085-2538
- VERRECCHIA FRANCK ET AL: "Transforming growth factor-beta and fibrosis." WORLD JOURNAL OF GASTROENTEROLOGY : WJG 14 JUN 2007 LNKD- PUBMED:17589920, vol. 13, no. 22, 14 June 2007 (2007-06-14) , pages 3056-3062, XP009137681 ISSN: 1007-9327
- BREITKOPF K ET AL: "279 BONE MORPHOGENETIC PROTEIN (BMP)-9: A NEW MEMBER OF THE TGF-beta SUPERFAMILY WHICH IS SECRETED BY ACTIVATED HEPATIC STELLATE CELLS", JOURNAL OF HEPATOLOGY, MUNKSGAARD INTERNATIONAL PUBLISHERS, COPENHAGEN, DK, vol. 50, 1 April 2009 (2009-04-01), page S110, XP026495892, ISSN: 0168-8278, DOI: 10.1016/S0168-8278(09)60281-6 [retrieved on 2009-04-01]
- ISMAIL MONA H ET AL: "Reversal of liver fibrosis.", SAUDI JOURNAL OF GASTROENTEROLOGY : OFFICIAL JOURNAL OF THE SAUDI GASTROENTEROLOGY ASSOCIATION JAN 2009, vol. 15, no. 1, January 2009 (2009-01), pages 72-79, ISSN: 1319-3767

## Description

### Background of the Invention

Fibrosis is a pathological process that refers to the aberrant formation or development of excess fibrous connective tissue by cells in an organ or tissue. Although processes related to fibrosis can occur as part of normal tissue formation or repair, dysregulation of these processes can lead to altered cellular composition and excess connective tissue deposition that progressively impairs tissue or organ function.

For example, more than five million people worldwide are afflicted with pulmonary fibrosis, which occurs when the air sacs of the lungs gradually become replaced by fibrotic tissue, causing an irreversible loss of the tissue's ability to transfer oxygen into the bloodstream. Additionally, hepatic fibrosis represents a major worldwide healthcare burden and results from excessive connective tissue formation in the liver which can lead to portal hypertension or even cirrhosis (see Murphy et al., Expert Opin Investig Drugs. 2002 Nov; 11(11):1575-85). Additionally, many other fibrotic disorders exist, including vascular fibrosis, pancreatic fibrosis, renal fibrosis, musculoskeletal fibrosis, cardiac fibrosis, skin fibrosis, eye fibrosis, progressive systemic sclerosis (PSS), chronic graft versus-host disease, Peyronie's disease, post-cystoscopic urethral stenosis, idiopathic and pharmacologically induced retroperitoneal fibrosis, mediastinal fibrosis, progressive massive fibrosis, proliferative fibrosis and neoplastic fibrosis.

Although therapeutic agents, such as anti-inflammatory drugs, are often used to treat fibrosis, such treatments can have low efficacy and undesirable side effects. Moreover, there are currently no wholly effective treatments or cures for fibrotic disorders. Accordingly, there is a great need in the art for moieties which can inhibit fibrosis and, therefore, can be used to treat or prevent fibrosis in a subject, as well as methods for diagnosing this debilitating disease.

### Summary of the Invention

The present invention provides anti-BMP9 antagonist antibodies for use in methods for treating or preventing fibrosis in a subject and methods for assessing whether a subject has or is at risk for developing a fibrotic disorder. The present invention is based, at least in part, on the demonstration by the present inventors that BMP9 and BMP 10 play a previously unknown role in the differentiation of fibroblasts and the excessive synthesis and accumulation of extracellular matrix and tissue remodeling by myofibroblasts during fibrosis.

In one aspect, the present invention provides methods for treating or preventing a fibrotic disorder in a subject. The methods include administering to a subject, *e.g.,* a human or an animal, an effective amount of a bone morphogenetic protein 9 (BMP9) antagonist antibody. In one embodiment, the fibrotic disorder includes, but is not limited to, vascular fibrosis, pulmonary fibrosis (*e.g.,* idiopathic pulmonary fibrosis), pancreatic fibrosis, liver fibrosis (*e.g*.,cirrhosis), renal fibrosis, musculoskeletal fibrosis, cardiac fibrosis (*e.g.,* endomyocardial fibrosis, idiopathic myocardiopathy), skin fibrosis *(e.g.,* scleroderma, post-traumatic, operative cutaneous scarring, keloids and cutaneous keloid formation), eye fibrosis (*e.g.,* glaucoma, sclerosis of the eyes, conjunctival and corneal scarring, and pterygium), progressive systemic sclerosis (PSS), chronic graft versus-host disease, Peyronie's disease, post-cystoscopic urethral stenosis, idiopathic and pharmacologically induced retroperitoneal fibrosis, mediastinal fibrosis, progressive massive fibrosis, proliferative fibrosis, and neoplastic fibrosis. In a particular embodiment, the fibrotic disorder is not myelofibrosis.

In a particular embodiment, the present invention provides an anti-BMP9 antagonist antibody for use in a method of treating or preventing a fibrotic disorder in a subject, comprising administering to the subject an effective amount of an anti-BMP9 , wherein the fibrotic disorder is selected from the group consisting of liver fibrosis, kidney fibrosis, heart fibrosis, skin fibrosis, and lung fibrosis.

In a further aspect, the present invention provides methods of assessing whether a subject has or is at risk of developing a fibrotic disorder. The methods include contacting a sample from a subject with a reagent able to detect BMP9 and detecting BMP9, wherein an elevated level of BMP9 (*e.g.,* at least 1.5, 2, 2.5, 3, 3.5, 4, 4.5, 5 -fold higher relative to the control) is an indication that the subject has or is at risk of developing a fibrotic disorder. In a particular embodiment, the method includes a further step of detecting the presence of additional fibrosis markers, *e.g.,* alpha smooth muscle actin, collagen type III cartilage oligomeric matrix protein, collagen type I, collagen type IV, fibroblast specific protein-1, fibronectin, serpinE1, periostin, IGFBP3, SPARC, CTGF, TGFb, Cyr61, and phospho smad 2/3.

In one embodiment, the sample includes cells or tissues obtained from the subject. In another embodiment, the sample is a fluid (*e.g.,* blood fluid, lymph, gynecological fluid, cystic fluid, ocular fluid, urine, and fluid collected by peritoneal rinsing) obtained from the subject.

The reagents encompassed by the methods of the invention include any known agent capable of detecting BMP9. In one embodiment, the reagent is an antibody. In another, the reagent is a nucleic acid. The reagents may also be labeled *(e.g.,* a radioisotope, a bioluminescent compound, a chemiluminescent compound, a fluorescent compound, a metal chelate, or an enzyme), so as to facilitate detection of BMP9.

In yet a further aspect, the present invention provides methods of assessing the efficacy of a treatment regimen for treating a fibrotic disorder in a subject. These methods include contacting a first sample obtained from the subject prior to administering at least a portion of the treatment regimen to the subject with a reagent able to detect BMP9, contacting a second sample obtained from the subject following administration of at least a portion of the treatment regimen with a reagent able to detect BMP9 or BMP10, comparing the levels of BMP9 from the first and second samples, wherein an elevated level of BMP9 present in the first sample, relative to the second sample, is an indication that the treatment regimen is efficacious for treating a fibrotic disorder in the subject. In a particular embodiment, the treatment regimen comprises administration of a BMP9 antagonist antibody.

Antibodies for use according to the invention include all known forms of antibodies having at least variable region sequences. For example, the antibody can be a murine, human, humanized, chimeric or bispecific monoclonal antibody. The antibody can be a Fab, Fab'2, ScFv, SMIP, affibody, avimer, versabody, nanobody, and a domain antibody and the antibody can be an IgG1, IgG2, IgG3, IgG4, IgM, IgA1, IgA2, IgAsec, IgD, or IgE antibody.

BMP9 antagonist antibodies used in the methods of the present invention can be administered via any suitable method including, but not limited to intravenous, intramuscular or subcutaneous administration.

Other features and advantages of the invention will be apparent from the following detailed description, and from the claims.

### Brief Description of the Drawing

**Figure 1** depicts the results of a high content immunostaining experiment leading to the identification of BMP9 (*i.e.,* GDF2) and BMP10 as therapeutic targets capable of treating fibrotic disorders.
**Figure 2** is a graph depicting BMP9 and BMP10 activity on myofibroblastic differentiation of fibroblasts in a High Throughput Screening (HTS) assay.
**Figure 3** is a graph depicting the upregulation of collagen type III, a known marker of fibrosis, after treatment of fibroblasts with recombinant BMP9 or BMP10.
**Figure 4** is a graph depicting the upregulation of alpha smooth actin, a known marker of fibrosis, after treatment of fibroblasts with recombinant BMP9 or BMP10.
**Figure 5** is a graph depicting upregulation of cartilage oligomeric matrix protein (COMP), a known marker of fibrosis, after treatment of fibroblasts with recombinant BMP9 or BMP10.
**Figure 6** is a graph depicting the effects of an anti-BMP9 monoclonal antibody and an ALK1-Fc soluble receptor on BMP9-stimulated BRE-luciferase activity in a reporter gene assay.

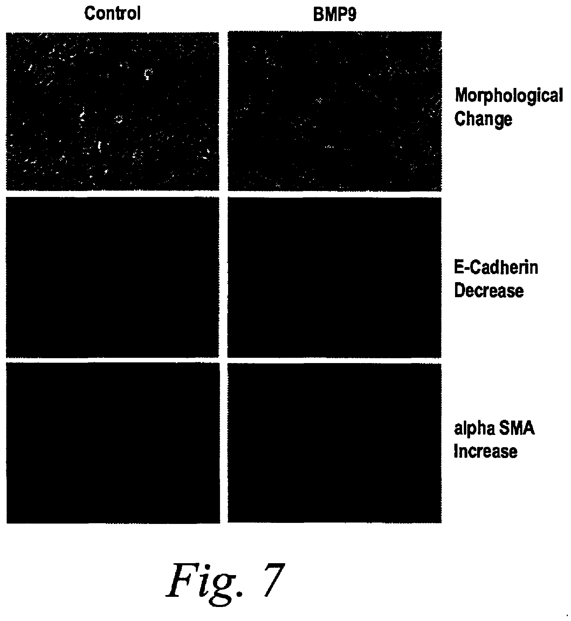

**Figure 7** depicts the morphological and immunohistochemical changes in Hep3B cells treated with or without BMP9 in an Epithelial-Mesenchymal Transdifferentiation (EMT) assay.
**Figures 8A-8D** are graphs depicting the effect of BMP9 on the expression of four different fibroblast markers (*i.e.,* αSMA, Col la1, fibroblast specific protein, and vimentin) in an EMT assay.
**Figure 9** depicts the effects of a BMP9 neutralizing monoclonal antibody and a soluble receptor (ALK1-Fc) on BMP9-induced FSP-1 expression in an EMT assay.

### Detailed Description of the Invention

The present invention provides the use of anti-BMP9 antagonistic antibodies for treating or preventing a fibrotic disorder in a subject and methods for assessing whether a subject has or is at risk for developing a fibrotic disorder. The present invention is based, at least in part, on the demonstration by the present inventors that BMP9 and BMP10 play a previously unknown role in the differentiation of fibroblasts into myofibroblasts during the development of fibrosis.

In order that the present invention may be more readily understood, certain terms are first defined. Additional definitions are provided throughout the detailed description.

### I. Definitions

As used herein, the term bone morphogenetic proteins (also referenced interchangeably herein as "BMPs") refers to a group of multi-functional growth factors and cytokines known for their ability to induce the formation of bone and cartilage. To date, approximately 20 BMPs have been identified. With the exception of bone morphogenetic protein 1 (BMP1), all of the bone morphogenetic proteins belong to the transforming growth factor beta (TGF β) superfamily (see Chen et al., Growth Factors. 2004 Dec;22(4):233-41).

As used herein, the term bone morphogenetic protein 9 (also referenced interchangeably herein as "BMP9", "BMP-9", "growth differentiation factor 2", "GDF-2", "GDF2," and "Growth/differentiation factor 2 precursor" refers to the art known member of the TGFβ/BMP superfamily that is known to be a potent inducer of osteoblast differentiation of mesenchymal stem cells (see Tang et al. (2008) J Cell Mol Med. [PMID: 19175684]). BMP9 has also been shown to be involved in the regulation of glucose metabolism, capable of reducing glycemia in diabetic mice, a differentiation factor for cholinergic neurons in the central nervous system, and to induce the expression of a hormone (hepcidin) that plays a role in iron homeostatis (David et al. 2008. Circ Res. Apr 25;102(8):914-22).

It was found that upon activation of hepatic stem cells, these rapidly secrete increasing amounts of BMP9, which then indusces Smad1 mediated signaling in hepatocytes, thus affecting expression of genes mediating profilation and/or fibrogenesis (Breitkopf et al. 2009, Journal of Hepatology, vol.50, 1 April, page S110). A representative BMP9 sequence, includes, but is not limited to, the sequence set forth below.
BMP9 / Growth Differentiation Factor 2 [Homo sapiens] (NP_057288) (SEQ ID NO:1) 
MCPGALWVALPLLSLLAGSLQGKPLQSWGRGSAGGNAHSPLGVPGGGLPE
HTFNLKMFLENVKVDFLRSLNLSGVPSQDKTRVEPPQYMIDLYNRYTSDKSTTPA
SNIVRSFSMEDAISITATEDFPFQKHILLFNISIPRHEQITRAELRLYVSCQNHVDPSH
DLKGSVVIYDVLDGTDAWDSATETKTFLVSQDIQDEGWETLEVSSAVKRWVRSD
STKSNKLEVTVESHRKGCDTLDISVPPGSRNLPFFVVFSNDHSSGTKETRLELRE
MISHEQESVLKKLSKDGSTEAGESSHEEDTDGHVAAGSTLARRKRSAGAGSHCQ
 
KTSLRVNFEDIGWDSWIIAPKEYEAYECKGGCFFPLADDVTPTKHAIVQTLVHLK
FPTKVGKACCVPTKLSPISVLYKDDMGVPTLKYHYEGMSVAECGCR
BMP9 / Growth Differentiation Factor 2 [Homo sapiens] (AF188285) (SEQ ID NO:2)

cggtccagcc cggcagcggg tgagagtggg tgctggccag gacggttcct tcagagcaaa
cagcagggag atgccggccc gctccttccc agctcctccc cgtgcccgct aacacagcac
ggccgcctgc agtctcctct ctgggtgatt gcgcgggcct aagatgtgtc ctggggcact
gtgggtggcc ctgcccctgc tgtccctgct ggctggctcc ctacagggga agccactgca
gagctgggga cgagggtctg ctgggggaaa cgcccacagc ccactggggg tgcctggagg
tgggctgcct gagcacacct tcaacctgaa gatgtttctg gagaacgtga aggtggattt
cctgcgcagc cttaacctga gtggggtccc ttcgcaggac aaaaccaggg tggagccgcc
gcagtacatg attgacctgt acaacaggta cacgtccgat aagtcgacta cgccagcgtc
caacattgtg cggagcttca gcatggaaga tgccatctcc ataactgcca cagaggactt
ccccttccag aagcacatct tgctcttcaa catctccatt cctaggcatg agcagatcac
cagagctgag ctccgactct atgtctcctg tcaaaatcac gtggacccct ctcatgacct
gaaaggaagc gtggtcattt atgatgttct ggatggaaca gatgcctggg atagtgctac
agagaccaag accttcctgg tgtcccagga cattcaggat gagggctggg agaccttgga
agtgtccagc gccgtgaagc gctgggtccg gtccgactcc accaagagca aaaataagct
ggaagtgact gtggagagcc acaggaaggg ctgcgacacg ctggacatca gtgtcccccc
aggttccaga aacctgccct tctttgttgt cttctccaat gaccacagca gtgggaccaa
ggagaccagg ctggagctga gggagatgat cagccatgaa caagagagcg tgctcaagaa
gctgtccaag gacggctcca cagaggcagg tgagagcagt cacgaggagg acacggatgg
ccacgtggct gcggggtcga ctttagccag gcggaaaagg agcgccgggg ctggcagcca
ctgtcaaaag acctccctgc gggtaaactt cgaggacatc ggctgggaca gctggatcat
tgcacccaag gagtatgaag cctacgagtg taagggcggc tgcttcttcc ccttggctga
cgatgtgacg ccgacgaaac acgctatcgt gcagaccctg gtgcatctca agttccccac
aaaggtgggc aaggcctgct gtgtgcccac caaactgagc cccatctccg tcctctacaa
ggatgacatg ggggtgccca ccctcaagta ccattacgag ggcatgagcg tggcagagtg
tgggtgcagg tagtatctgc ctgcggggct ggggaggcag gccaaagggg ctccacatga
gaggtcctgc atgcccctgg gcacaacaag gactgattca atctgcatgc cagcctggag
gaggaaaggg agcctgctct ccctccccac accccaccca aagcatacac cgctgagctc
aactgccagg gaaggctaag gaaatgggga tttgagcaca acaggaaagc ctgggagggt
tgttgggatg caaggaggtg atgaaaagga gacaggggga aaaataatcc atagtcagca
gaaaacaaca gcagtgagcc agaggagcac aggcgggcag gtcactgcag agactgatgg
aagttagaga ggtggaggag gccagctcgc tccaaaaccc ttggggagta gagggaagga
gcaggccgcg tgtcacaccc atcattgtat gttatttccc acaacccagt tggagggcca
tggcttccaa tttagagacc cg

The murine and other animal BMP9 molecules are known in the art (see, for example, NP_062379 for murine BMP9 and NP_001099566 for rat BMP9).

As used herein, the term bone morphogenetic protein 10 (also referenced interchangeably herein as "BMP10", "BMP-10", "MGC126783", and "Bone morphogenetic protein 10 precursor" refers to art known member of the TGFβ/BMP superfamily. It has been suggested that BMP10 is an essential component in modulating cardiomyocyte proliferation and maturation during cardiac ventricular development. (Chen et al.,(2004) Development.131(9):2219-31 and Neuhaus et al., (1999) Mech Dev., 80(2):181-4).

A representative BMP10 sequence, includes, but is not limited to, the sequence set forth below.
Bone Morphogenetic Protein 10 Preproprotein [Homo sapiens](NP_055297) (SEQ ID NO:3) 
MGSLVLTLCALFCLAAYLVSGSPIMNLEQSPLEEDMSLFGDVFSEQDGVDFNTLL
QSMKDEFLKTLNLSDIPTQDSAKVDPPEYMLELYNKFATDRTSMPSANIIRSFKNE
DLFSQPVSFNVSIPHHEEVIMAELRLYTLVQRDRMIYDGVDRKITIFEVLESKGDN
EGERNMLVLVSGEIYGTNSEWETFDVTDAIRRWQKSGSSTHQLEVHIESKHDEAE
DASSGRLEIDTSAQNKHNPLLIVFSDDQSSDKERKEELNEMISHEQLPELDNLGLD
SFSSGPGEEALLQMRSNIIYDSTARIRRNAKGNYCKRTPLYIDFKEIGWDSWIIAPP
GYEAYECRGVCNYPLAEHLTPTKHAIIQALVHLKNSQKASKACCVPTKLEPISILY
LDKGVVTYKFKYEGMAVSECGCR
Bone Morphogenetic Protein 10 [Homo sapiens](NM_014482) (SEQ ID NO:4)

ggggagagga agagtggtag ggggagggag agagagagga agagtttcca aacttgtctc
cagtgacagg agacatttac gttccacaag ataaaactgc cacttagagc ccagggaagc
taaaccttcc tggcttggcc taggagctcg agcggagtca tgggctctct ggtcctgaca
ctgtgcgctc ttttctgcct ggcagcttac ttggtttctg gcagccccat catgaaccta
gagcagtctc ctctggaaga agatatgtcc ctctttggtg atgttttctc agagcaagac
ggtgtcgact ttaacacact gctccagagc atgaaggatg agtttcttaa gacactaaac
ctctctgaca tccccacgca ggattcagcc aaggtggacc caccagagta catgttggaa
ctctacaaca aatttgcaac agatcggacc tccatgccct ctgccaacat cattaggagt
ttcaagaatg aagatctgtt ttcccagccg gtcagtttta atgggctccg aaaatacccc
ctcctcttca atgtgtccat tcctcaccat gaagaggtca tcatggctga acttaggcta
tacacactgg tgcaaaggga tcgtatgata tacgatggag tagaccggaa aattaccatt
tttgaagtgc tggagagcaa aggggataat gagggagaaa gaaacatgct ggtcttggtg
tctggggaga tatatggaac caacagtgag tgggagactt ttgatgtcac agatgccatc
agacgttggc aaaagtcagg ctcatccacc caccagctgg aggtccacat tgagagcaaa
cacgatgaag ctgaggatgc cagcagtgga cggctagaaa tagataccag tgcccagaat
aagcataacc ctttgctcat cgtgttttct gatgaccaaa gcagtgacaa ggagaggaag
gaggaactga atgaaatgat ttcccatgag caacttccag agctggacaa cttgggcctg
gatagctttt ccagtggacc tggggaagag gctttgttgc agatgagatc aaacatcatc
tatgactcca ctgcccgaat cagaaggaac gccaaaggaa actactgtaa gaggaccccg
ctctacatcg acttcaagga gattgggtgg gactcctgga tcatcgctcc gcctggatac
gaagcctatg aatgccgtgg tgtttgtaac taccccctgg cagagcatct cacacccaca
aagcatgcaa ttatccaggc cttggtccac ctcaagaatt cccagaaagc ttccaaagcc
tgctgtgtgc ccacaaagct agagcccatc tccatcctct atttagacaa aggcgtcgtc
acctacaagt ttaaatacga aggcatggcc gtctccgaat gtggctgtag atagaagaag
agtcctatgg cttatttaat aactgtaaat gtgtatattt ggtgttccta tttaatgaga
ttatttaata agggtgtaca gtaatagagg cttgctgcct tcaggaaatg gacaggtcag
tttgttgtag gaaatgcata tttt

The murine and other animal BMP10 molecules are known in the art (see, for example, NP_033886 for murine BMP10).

As used herein, the term "antagonist" refers to any moiety which downregulates BMP9 and/or BMP10 activity, including agents which downregulate BMP9 and/or BMP10 expression or inhibit BMP9 and/or BMP10 function.

As used herein, the term "downregulates" refers to any statistically significant decrease in a biological activity and/or expression of BMP9 and/or BMP10, including full blocking of the activity (*i.e.,* complete inhibition) and/or expression. For example, "downregulation" can refer to a decrease of about 10%, 20%, 30%, 40%, 50%, 60%, 70%, 80%, 90%, or 100% in BMP9 and/or BMP10 activity and/or expression.

As used herein, the term "inhibit" or "inhibiting" fibrosis refers to any statistically significant decrease in a biological activity and/or expression of BMP9 and/or BMP10, including full blocking of the activity and/or expression. For example, "inhibition" can refer to a decrease of about 10%, 20%, 30%, 40%, 50%, 60%, 70%, 80%, 90%, or 100% in BMP9 and/or BMP10 activity and/or expression.

As used herein, the term "fibrosis" refers to the aberrant formation or development of excess fibrous connective tissue by cells in an organ or tissue. Although processes related to fibrosis can occur as part of normal tissue formation or repair, dysregulation of these processes can lead to altered cellular composition and excess connective tissue deposition that progressively impairs to tissue or organ function. There are several types of fibrosis, for example, cystic fibrosis of the pancreas and lungs, injection fibrosis, which can occur as a complication of intramuscular injections, especially in children, endomyocardial fibrosis , idiopathic pulmonary fibrosis of the lung, mediastinal fibrosis, myleofibrosis, retroperitoneal fibrosis, progressive massive fibrosis, a complication of coal workers' pneumoconiosis, and nephrogenic systemic fibrosis.

As used herein, the terms "fibrotic disorder", "fibrotic condition," and "fibrotic disease," are used interchangeably to refer to a disorder, condition or disease characterized by fibrosis. Examples of fibrotic disorders include, but are not limited to vascular fibrosis, pulmonary fibrosis (*e.g.,* idiopathic pulmonary fibrosis), pancreatic fibrosis, liver fibrosis (*e.g*.,cirrhosis), renal fibrosis, musculoskeletal fibrosis, cardiac fibrosis (*e.g.,* endomyocardial fibrosis, idiopathic myocardiopathy), skin fibrosis (*e.g.,* scleroderma, post-traumatic, operative cutaneous scarring, keloids and cutaneous keloid formation), eye fibrosis (*e.g.,* glaucoma, sclerosis of the eyes, conjunctival and corneal scarring, and pterygium), progressive systemic sclerosis (PSS), chronic graft versus-host disease, Peyronie's disease, post-cystoscopic urethral stenosis, idiopathic and pharmacologically induced retroperitoneal fibrosis, mediastinal fibrosis, progressive massive fibrosis, proliferative fibrosis and neoplastic fibrosis.

As used herein, the term "cell" refers to any cell prone to undergoing a fibrotic response, including, but not limited to, individual cells, tissues, and cells within tissues and organs. The term cell, as used herein, includes the cell itself, as well as the extracellular matrix (ECM) surrounding a cell. For example, inhibition of the fibrotic response of a cell, includes, but is not limited to the inhibition of the fibrotic response of one or more cells within the lung (or lung tissue); one or more cells within the liver (or liver tissue); one or more cells within the kidney (or renal tissue); one or more cells within muscle tissue; one or more cells within the heart (or cardiac tissue); one or more cells within the pancreas; one or more cells within the skin; one or more cells within the bone, one or more cells within the vasculature, one or more stem cells, or one or more cells within the eye.

As used herein, the term "Epithelial-Mesenchymal Transition" (EMT) refers to the conversion from an epithelial to a mesenchymal phenotype, which is a normal process of embryonic development. EMT is also the process whereby injured epithelial cells that function as ion and fluid transporters become matrix remodeling mesenchymal cells. In carcinomas, this transformation results in altered cell morphology, the expression of mesenchymal proteins and increased invasiveness. The criteria for defining EMT *in vitro* involve the loss of epithelial cell polarity, the separation into individual cells and subsequent dispersion after the acquisition of cell motility (See Vincent-Salomon et al., Breast Cancer Res. 2003; 5(2): 101-106). Classes of molecules that change in expression, distribution, and/or function during EMT, and that are causally involved, include growth factors (*e.g.,* transforming growth factor (TGF)-β, wnts), transcription factors (*e.g.,* snails, SMAD, LEF, and nuclear β-catenin), molecules of the cell-to-cell adhesion axis (cadherins, catenins), cytoskeletal modulators (Rho family), and extracellular proteases (matrix metalloproteinases, plasminogen activators) (see Thompson et al., Cancer Research 65, 5991-5995, July 15, 2005).

### II. Methods of Treatment or Prevention of Fibrotic Disorders

The present invention provides novel methods of treating and/or preventing a fibrotic disorder in a subject. The methods include administering to a subject an effective amount of a BMP9 antagonist, thereby treating and/or preventing a fibrotic disorder in a subject.

The terms "treat," "treated," "treating," and "treatment," as used herein, refer to the therapeutic measures described herein. The methods of "treatment" include administration of a BMP9 antagonist antibody to a subject in order to cure, reduce the severity of, or ameliorate one or more symptoms of a fibrotic disease or condition, in order to prolong the health or survival of a subject beyond that expected in the absence of such treatment. For example, "treatment" includes the alleviation of a fibrotic disease symptom (*e.g.,* shortness of breath, fatigue, cough, weight loss, loss of appetite associated with pulmonary fibrosis or anorexia, fatigue, weight loss, portal hypertension and ascites associated with liver fibrosis) in a subject by at least 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95% or more.

The terms "prevent," "prevented," "preventing", and "prevention," as used herein refer to the preventative measures described herein. The methods of "prevention" include administration of a BMP9 antagonist antibody to a subject in order to delay, prevent or preclude one or more symptoms of a disease or condition. For example, "prevention" includes a delayed onset or inhibition of a fibrotic disease symptom (*e.g.,* shortness of breath, fatigue, cough, weight loss, loss of appetite associated with pulmonary fibrosis or anorexia, fatigue, weight loss, portal hypertension and ascites associated with liver fibrosis) in a subject by at least 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95% or more.

The terms "patient" or "subject" as used herein is intended to include human and veterinary patients. In a particular embodiment, the subject is a human. The term "non-human animal" includes all vertebrates, *e.g.,* mammals and non-mammals, such as non-human primates, mice, rabbits, sheep, dog, cow, chickens, amphibians, and reptiles.

### A. Indications

The methods of the present invention can be used to treat and/or prevent fibrotic disorders. Exemplary types of fibrotic disorders include, but are not limited to, vascular fibrosis, pulmonary fibrosis (*e.g.,* idiopathic pulmonary fibrosis), pancreatic fibrosis, liver fibrosis *(e.g.,* cirrhosis), renal fibrosis, musculoskeletal fibrosis, cardiac fibrosis (*e.g.,* endomyocardial fibrosis, idiopathic myocardiopathy), skin fibrosis (*e.g.,* scleroderma, post-traumatic, operative cutaneous scarring, keloids and cutaneous keloid formation), eye fibrosis (*e.g.,* glaucoma, sclerosis of the eyes, conjunctival and corneal scarring, and pterygium), progressive systemic sclerosis (PSS), chronic graft versus-host disease, Peyronie's disease, post-cystoscopic urethral stenosis, idiopathic and pharmacologically induced retroperitoneal fibrosis, mediastinal fibrosis, progressive massive fibrosis, proliferative fibrosis, neoplastic fibrosis, Dupuytren's disease, strictures, and radiation induced fibrosis. In a particular embodiment, the fibrotic disorder is not myelofibrosis.

### B. Combination Therapies

The present invention contemplates the use of BMP9 and antagonists in combination with one or more other therapeutic modalities. Thus, in addition to the use of BMP9 antagonist antibody, one may also administer to the subject one or more "standard" therapies for treating fibrotic disorders. For example, the antagonists can be administered in combination with *(i.e.,* together with or linked to *(i.e.,* an immunoconjugate)) cytotoxins, immunosuppressive agents, radiotoxic agents, and/or therapeutic antibodies. Particular co-therapeutics contemplated by the present invention include, but are not limited to, steroids (*e.g.,* corticosteroids, such as Prednisone), immune-suppressing and/or anti-inflammatory agents (*e.g.,* gamma-interferon, cyclophosphamide, azathioprine, methotrexate, penicillamine, cyclosporine, colchicines, antithymocyte globulin, mycophenolate mofetil, and hydroxychloroquine), cytotoxic drugs, calcium channel blockers (*e.g.,* nifedipine), angiotensin converting enzyme inhibitors (ACE) inhibitors, para-aminobenzoic acid (PABA), dimethyl sulfoxide, transforming growth factor-beta (TGF-β) inhibitors, interleukin-5 (IL-5) inhibitors, and pan caspase inhibitors.

Additional anti-fibrotic agents that may be used in combination with a BMP9 antagonist antibody include, but are not limited to, lectins (as described in, for example, U.S. Patent No.: 7,026,283, as well as the anti-fibrotic agents described by Wynn et al (Journal Clin. Invest. Vol 117 Number 3, March 2007, p524. For example, additional anti-fibrotic agents and therapies include, but are not limited to, various anti-inflammatory/ immunosuppressive/ cytotoxic drugs (including colchicine, azathioprine, cyclophosphamide, prednisone,thalidomide, pentoxifylline, and theophylline), TGF-β signaling modifiers (including relaxin, SMAD7, HGF, and BMP7, as well as TGF-β1, TGFβRI, TGFβRII, EGR-1, and CTGF inhibitors), cytokine and cytokine receptor antagonists (inhibitors of IL-1β, IL-5, IL-6, IL-13, IL-21, IL-4R, IL-13Rα1, GM-CSF, TNF-α, oncostatin M, WISP-1, and PDGFs), cytokines and chemokines (IFN-γ, IFN-α/β, IL-12, IL-10, HGF, CXCL10, and CXCL11), chemokine antagonists (inhibitors of CXCL1, CXCL2, CXCL12, CCL2, CCL3, CCL6, CCL17, and CCL18), chemokine receptor antagonists (inhibitors of CCR2, CCR3, CCR5, CCR7, CXCR2, and CXCR4), TLR antagonists (inhibitors of TLR3, TLR4, and TLR9), Angiogenesis antagonists (VEGF-specific antibodies and adenosine deaminase replacement therapy), Antihypertensive drugs (beta blockers and inhibitors of ANG II, ACE, and aldosterone), Vasoactive substances (ET-1 receptor antagonists and bosetan), Inhibitors of the enzymes that synthesize and process collagen (inhibitors of prolyl hydroxylase), B cell antagonists (rituximab), Integrin/adhesion molecule antagonists (molecules that block α1β1 and αvβ6 integrins, as well as inhibitors of integrin linked kinase, and antibodies specific for ICAM-1 and VCAM-1), proapoptotic drugs that target myofibroblasts, MMP inhibitors (inhibitors of MMP2, MMP9, and MMP12), and TIMP inhibitors (antibodies specific for TIMP-1).

The BMP9 antagonist antibody and the co-therapeutic agent or co-therapy can be administered in the same formulation or separately. In the case of separate administration, the BMP9 antagonist antibody can be administered before, after or concurrently with the co-therapeutic or co-therapy. One agent may precede or follow administration of the other agent by intervals ranging from minutes to weeks. In embodiments where two or more different kinds of therapeutic agents are applied separately to a subject, one would generally ensure that a significant period of time did not expire between the time of each delivery, such that these different kinds of agents would still be able to exert an advantageously combined effect on the target tissues or cells.

In one embodiment, the BMP9 antagonist antibody (*e.g.,* an anti-BMP9 or anti-antibody) may be linked to a second binding molecule, such as an antibody (*i.e*., thereby forming a bispecific molecule) or other binding agent that, for example, binds to a different target or a different epitope on BMP9 or BMP10. Examples of additional therapeutic agents that can be used in combination therapy with the antagonists disclosed herein are described in greater detail below in the section on immunoconjugates.

### C. Dosages/ Amounts

The terms "effective amount" and "therapeutically effective amount" as used herein, refer to that amount of a BMP9 antagonist antibody which is sufficient to effect treatment or prevention of a fibrotic disorder, as described herein, when administered to a subject. A therapeutically effective amount will vary depending upon the subject and the severity of the fibrotic disorder being treated, the weight and age of the subject, the manner of administration and the like, which can readily be determined by one of ordinary skill in the art. The BMP9 antagonist antibody dosages for administration can range from, for example, about 1 ng to about 10,000 mg, about 5 ng to about 9,500 mg, about 10 ng to about 9,000 mg, about 20 ng to about 8,500 mg, about 30 ng to about 7,500 mg, about 40 ng to about 7,000 mg, about 50 ng to about 6,500 mg, about 100 ng to about 6,000 mg, about 200 ng to about 5,500 mg, about 300 ng to about 5,000 mg, about 400 ng to about 4,500 mg, about 500 ng to about 4,000 mg, about 1 µg to about 3,500 mg, about 5 µg to about 3,000 mg, about 10 µg to about 2,600 mg, about 20 µg to about 2,575 mg, about 30 µg to about 2,550 mg, about 40 µg to about 2,500 mg, about 50 µg to about 2,475 mg, about 100 µg to about 2,450 mg, about 200 µg to about 2,425 mg, about 300 µg to about 2,000, about 400 µg to about 1,175 mg, about 500 µg to about 1,150 mg, about 0.5 mg to about 1,125 mg, about 1 mg to about 1,100 mg, about 1.25 mg to about 1,075 mg, about 1.5 mg to about 1,050 mg, about 2.0 mg to about 1,025 mg, about 2.5 mg to about 1,000 mg, about 3.0 mg to about 975 mg, about 3.5 mg to about 950 mg, about 4.0 mg to about 925 mg, about 4.5 mg to about 900 mg, about 5 mg to about 875 mg, about 10 mg to about 850 mg, about 20 mg to about 825 mg, about 30 mg to about 800 mg, about 40 mg to about 775 mg, about 50 mg to about 750 mg, about 100 mg to about 725 mg, about 200 mg to about 700 mg, about 300 mg to about 675 mg, about 400 mg to about 650 mg, about 500 mg, or about 525 mg to about 625 mg, of a BMP9 antagonist antibody. Dosage regimens may be adjusted to provide the optimum therapeutic response. An effective amount is also one in which any toxic or detrimental effects (*i.e.,* side effects) of a BMP9 antagonist antibody are minimized and/or outweighed by the beneficial effects.

Actual dosage levels of the BMP9 antagonist antibody used in the methods of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular BMP9 antagonist antibody employed, or the ester, salt or amide thereof, the route of administration, the time of administration, the rate of excretion of the particular antagonist being employed, the duration of the treatment, other drugs, compounds and/or materials used in combination with the particular antagonist employed, the age, sex, weight, condition, general health and prior medical history of the patient being treated, and like factors well known in the medical arts. A physician or veterinarian having ordinary skill in the art can readily determine and prescribe the effective amount of the antagonist required. For example, the physician or veterinarian could start doses of the antagonist at levels lower than that required in order to achieve the desired therapeutic effect and gradually increase the dosage until the desired effect is achieved. In general, a suitable daily dose of a BMP9 antagonist antibody will be that amount which is the lowest dose effective to produce a therapeutic effect. Such an effective dose will generally depend upon the factors described above. It is preferred that administration be intravenous, intramuscular, intraperitoneal, or subcutaneous, preferably administered proximal to the site of the target. If desired, the effective daily dose of a BMP9 antagonist antibody may be administered as two, three, four, five, six or more sub-doses administered separately at appropriate intervals throughout the day, optionally, in unit dosage forms. While it is possible for a BMP9 antagonist antibody of the present invention to be administered alone, it is preferable to administer the antagonist as a pharmaceutical formulation (composition).

Dosage regimens are adjusted to provide the optimum desired response *(e.g.,* a therapeutic response). For example, a single bolus may be administered, several divided doses may be administered over time or the dose may be proportionally reduced or increased as indicated by the exigencies of the therapeutic situation. For example, the BMP9 antagonist antibody used in the methods of the present invention may be administered once or twice weekly by subcutaneous injection or once or twice monthly by subcutaneous injection.

It is especially advantageous to formulate parenteral BMP9 antagonist antibody in dosage unit form for ease of administration and uniformity of dosage. Dosage unit form as used herein refers to physically discrete units suited as unitary dosages for the subjects to be treated; each unit contains a predetermined quantity of active antagonist calculated to produce the desired therapeutic effect in association with the required pharmaceutical carrier. The specification for the dosage unit forms are dictated by and directly dependent on (a) the unique characteristics of the active antagonist and the particular therapeutic effect to be achieved, and (b) the limitations inherent in the art of compounding such an active antagonist for the treatment of sensitivity in subjects.

### D. Methods of Administration and Formulations

To administer a BMP9 antagonist antibody used in the methods of the present invention by certain routes of administration, it may be necessary to include the antagonist in a formulation suitable for preventing its inactivation. For example, the BMP9 antagonist antibody may be administered to a subject in an appropriate carrier, for example, liposomes, or a diluent. Pharmaceutically acceptable diluents include saline and aqueous buffer solutions. Liposomes include water-in-oil-in-water CGF emulsions, as well as conventional liposomes (Strejan et al. (1984) J. Neuroimmunol. 7:27).

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active BMP9 antagonist antibody, use thereof in a pharmaceutical compositions is contemplated. Supplementary active compounds can also be incorporated with the BMP9 antagonist antibody.

Therapeutic BMP9 antagonist antibody typically must be sterile and stable under the conditions of manufacture and storage. The antagonist can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including an agent that delays absorption, for example, monostearate salts and gelatin.

Sterile injectable solutions can be prepared by incorporating the active antagonist in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

BMP9 antagonist antibody that can be used in the methods of the present invention include those suitable for oral, nasal, topical (including buccal and sublingual), rectal, vaginal and/or parenteral administration. The formulations may conveniently be presented in unit dosage form and may be prepared by any methods known in the art of pharmacy. The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the antagonist which produces a therapeutic effect. Generally, out of one hundred per cent, this amount will range from about 0.001 per cent to about ninety percent of active ingredient, preferably from about 0.005 per cent to about 70 per cent, most preferably from about 0.01 per cent to about 30 per cent.

The phrases "parenteral administration" and "administered parenterally", as used herein, means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Examples of suitable aqueous and nonaqueous carriers which may be employed along with the BMP9 antagonist antibody utilized in the methods of the present invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

The BMP9 antagonist antibodymay also be administered with adjuvants such as preservatives, wetting agents, emulsifying agents and dispersing agents. Prevention of presence of microorganisms may be ensured both by sterilization procedures and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

When the BMP9 antagonist antibody used in the methods of the present invention are administered to humans and animals, they can be given alone or as a pharmaceutical antagonist containing, for example, 0.001 to 90% (more preferably, 0.005 to 70%, such as 0.01 to 30%) of active ingredient in combination with a pharmaceutically acceptable carrier.

The BMP9 antagonist antibody can be administered with medical devices known in the art. For example, in a preferred embodiment, an antagonist can be administered with a needleless hypodermic injection device, such as the devices disclosed in U.S. Patent Nos. 5,399,163, 5,383,851, 5,312,335, 5,064,413, 4,941,880, 4,790,824, or 4,596,556. Examples of well-known implants and modules useful in the present invention include: U.S. Patent No. 4,487,603, which discloses an implantable micro-infusion pump for dispensing medication at a controlled rate; U.S. Patent No. 4.,486,194, which discloses a therapeutic device for administering medications through the skin; U.S. Patent No. 4,447,233, which discloses a medication infusion pump for delivering medication at a precise infusion rate; U.S. Patent No. 4,447,224, which discloses a variable flow implantable infusion apparatus for continuous drug delivery; U.S. Patent No. 4,439,196, which discloses an osmotic drug delivery system having multi-chamber compartments; and U.S. Patent No. 4,475,196, which discloses an osmotic drug delivery system. Many other such implants, delivery systems, and modules are known to those skilled in the art.

In certain embodiments, antagonists can be formulated to ensure proper distribution *in vivo.* For example, the blood-brain barrier (BBB) excludes many highly hydrophilic compounds. To ensure that the BMP9 antagonist antibody cross the BBB (if desired), they can be formulated, for example, in liposomes. For methods of manufacturing liposomes, see, *e.g.,* U.S. Patents 4,522,811; 5,374,548; and 5,399,331. The liposomes may comprise one or more moieties which are selectively transported into specific cells or organs, thus enhance targeted drug delivery (*see, e.g.,* V.V. Ranade (1989) J. Clin. Pharmacol. 29:685). Exemplary targeting moieties include folate or biotin (see, *e.g.,* U.S. Patent 5,416,016 to Low et al.); mannosides (Umezawa et al., (1988) Biochem. Biophys. Res. Commun. 153:1038); antibodies (P.G. Bloeman et al. (1995) FEBS Lett. 357:140; M. Owais et al. (1995) Antimicrob. Agents Chemother. 39:180); surfactant protein A receptor (Briscoe et al. (1995) Am. J. Physiol. 1233:134), different species of which may comprise the formulations of the inventions, as well as components of the invented molecules; p120 (Schreier et al. (1994) J. Biol. Chem. 269:9090); see also K. Keinanen; M.L. Laukkanen (1994) FEBS Lett. 346:123; J.J. Killion; I.J. Fidler (1994) Immunomethods 4:273.

### III. Diagnostic Methods

The present invention also provides novel methods for assessing whether a subject has or is at risk of developing a fibrotic disorder. Individuals suspected of having a fibrotic disorder would benefit from early detection, so that fibrotic disease progression can be retarded or even halted. The methods include assessing whether a subject has or is at risk of developing a fibrotic disorder comprising contacting a sample from a subject with a reagent able to detect BMP9 and detecting BMP9 or BMP10, wherein an elevated level of BMP9 relative to a control is an indication that the subject has or is at risk of developing a fibrotic disorder.

The invention further provides methods for determining or predicting the efficacy of a treatment regimen for treating a fibrotic disorder. These methods include assessing the efficacy of a treatment regimen for treating a fibrotic disorder in a subject, the method comprising: a) contacting a first sample obtained from the subject prior to administering at least a portion of the treatment regimen to the subject with a reagent able to detect BMP9 or BMP10; b) contacting a second sample obtained from the subject following administration of at least a portion of the treatment regimen with a reagent able to detect BMP9 or BMP10; and c) comparing the levels of BMP9 from the first and second samples, wherein an elevated level of BMP9 present in the first sample, relative to the second sample, is an indication that the treatment regimen is efficacious for treating a fibrotic disorder in the subject.

In a particular embodiment, the method can include a further step of detecting an additional marker of fibrosis, including, but not limited to, alpha smooth muscle actin, collagen type III, cartilage oligomeric matrix protein, Collagen type I, collagen type IV, fibroblast specific protein-1, fibronectin, serpinE1, periostin, IGFBP3, SPARC, CTGF, TGFb, Cyr61, and phospho smad 2/3, using assays well known in the art. Such assays include, but are not limited to, immunological methods for detection of proteins, protein purification methods, protein function or activity assays, nucleic acid hybridization methods, nucleic acid reverse transcription methods, and nucleic acid amplification methods, ELISA, immunoblotting, Western blotting, Northern blotting, electron microscopy, and Southern blotting. In a particular embodiment, the method can further include detecting fibrosis using biopsies, such as liver biopsies, or commercially available devices, such as Fibroscan (available from London, UK) or Fibrotest (available from France, Europe).

As used herein, the term "sample" includes any body fluid *(e.g.,* blood fluids, lymph, gynecological fluids, cystic fluid, urine, ocular fluids and fluids collected by peritoneal rinsing), or a cell from a subject. Normally, the tissue or cell will be removed from the patient, but *in vivo* diagnosis is also contemplated. Other patient samples, include tear drops, serum, cerebrospinal fluid, feces, sputum and cell extracts.

As used herein, the term "reagent able to detect BMP9 and/or BMP10" includes any agent capable of binding specifically with BMP9 and and transforming BMP9 and/ into a detectable moiety. Suitable reagents include antibodies, antibody derivatives, antibody fragments, and the like. Suitable reagents for binding with a BMP9 and/ nucleic acid (*e.g.* a genomic DNA, an mRNA, a spliced mRNA, a cDNA, or the like) include complementary nucleic acids. For example, the nucleic acid reagents may include oligonucleotides (labeled or non-labeled) fixed to a substrate, labeled oligonucleotides not bound with a substrate, pairs of PCR primers, molecular beacon probes, and the like.

As used herein, the term "control" can be the level of BMP9 and/ in a sample from a subject not suffering from fibrosis. It can be the same type of sample as the test sample or different. For example, if the sample from the subject being tested is a liver sample *(e.g.,* a cell, a collection of cells, or tissue obtained from a liver biopsy), then the control sample can also be a liver sample from a subject not suffering from a fibrotic disorder. Alternatively, the control sample can be of a different type, *e.g.,* it can be a blood sample from a subject not suffering from a fibrotic disorder. In other embodiments, the control sample can be a collection of samples from a subject not having a fibrotic disorder or a sample from a collection of subjects not have a fibrotic disorder.

As used herein, "an aberrant level" of BMP9 and/ is any level of BMP9 and/ that differs from the control level of BMP9 and/or BMP10, *e.g.,* significantly higher or elevated levels, or significantly lower or depressed levels.

As used herein, a "higher level," "elevated level," or "increased level" of BMP9 and/ refers to a level that is elevated relative to a suitable control. Preferably, the differential from the suitable control is greater than the standard error of the assay employed to assess the level. Moreover, the elevated level is preferably at least twice, and more preferably three, four, five, six, seven, eight, nine or ten times the level of BMP9 and/ in a suitable control (*e.g.,* a sample from a subject not having a fibrotic disease or the average level of BMP9 and/ in several control samples or other suitable benchmark).

As used herein, a "depressed level," "lower level" or "decreased level" of a BMP9 and/ refers to a level that is decreased relative to a suitable control. Preferably, the differential from the suitable control is greater than the standard error of the assay employed to assess the level. The decreased level preferably is at least twice, and more preferably three, four, five, six, seven, eight, nine or ten times lower than the level of the suitable control (*e.g.,* level in a healthy subject not having a fibrotic disease or the average level of BMP9 and/ in several control samples or other suitable benchmark).

As used herein, the terms "efficacious" and "efficacy" refers to the likelihood that a treatment regimen will treat a fibrotic disorder in a subject. For example, a treatment regimen is deemed "efficacious" and considered a viable treatment option if the treatment leads to an alleviation of the fibrotic disease symptoms (*e.g.,* shortness of breath, fatigue, cough, weight loss, loss of appetite associated with pulmonary fibrosis or anorexia, fatigue, weight loss, portal hypertension and ascites associated with liver fibrosis) in a subject by at least 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95% or more.

### A. Assays

The presence, absence, and/or level of BMP9 and/ in a biological sample obtained from a subject may be assessed by any of a wide variety of *in vitro* and *in vivo* techniques and methods, which transform BMP9 within the sample into a moiety that can be detected and quantified. Non-limiting examples of such methods include analyzing the sample using immunological methods for detection of proteins, protein purification methods, protein function or activity assays, nucleic acid hybridization methods, nucleic acid reverse transcription methods, and nucleic acid amplification methods, enzyme linked immunosorbent assays (ELISAs), immunoblotting, Western blotting, Northern blotting, electron microscopy, mass spectrometry, immunoprecipitations, immunofluorescence, Southern hybridizations and the like. Such techniques, as well as others described herein, can also be used to detect additional fibrotic markers, including, but not limited to alpha smooth muscle actin, collagen type III, cartilage oligomeric matrix protein, Collagen type I, collagen type IV, fibroblast specific protein-1, fibronectin, serpinE1, periostin, IGFBP3, SPARC, CTGF, TGFb, Cyr61, and phospho smad 2/3, where applicable.

In one embodiment, the presence, absence, and/or level BMP9 and/ in a sample can be assessed using a reagent, such as an antibody (*e.g.,* a radio-labeled, chromophore-labeled, fluorophore-labeled, or enzyme-labeled antibody), an antibody derivative (*e.g.,* an antibody conjugated with a substrate or with the protein or ligand of a protein-ligand pair (*e.g.* biotin-streptavidin)), or an antibody fragment (*e.g.,* a single-chain antibody or an isolated antibody hypervariable domain) which binds specifically to and transforms the biomarker, *e.g.,* BMP9 and/or BMP10, in a sample into a detectable molecule.

The term "labeled", with regard to the antibody, is intended to encompass direct labeling of the antibody by coupling (*i.e.,* physically linking) a detectable substance to the antibody, as well as indirect labeling of the antibody by reactivity with another reagent that is directly labeled. Examples of indirect labeling include detection of a primary antibody using a fluorescently labeled secondary antibody, such that it can be detected with fluorescently labeled streptavidin.

In another embodiment, the presence, absence, and/or level of BMP9 and/ is assessed using a nucleic acid. For example, in one embodiment, the presence, absence, and/or level of BMP9 and/ is assessed using a nucleic acid probe.

The term "probe", as used herein, refers to any molecule that is capable of selectively binding to BMP9 and/or BMP10. Probes can be synthesized by one of skill in the art, or derived from appropriate biological preparations. Probes may be specifically designed to be labeled. Examples of molecules that can be utilized as probes include, but are not limited to, RNA, DNA, proteins, antibodies, and organic molecules.

Isolated mRNA can be used in hybridization or amplification assays that include, but are not limited to, Southern or Northern analyses, polymerase chain reaction analyses and probe arrays. One method for the detection of mRNA levels involves contacting the isolated mRNA with a nucleic acid molecule (probe) that can hybridize to BMP9 and/ mRNA. The nucleic acid probe can be, for example, a full-length cDNA, or a portion thereof, such as an oligonucleotide of at least 7, 15, 30, 50, 100, 250 or 500 nucleotides in length and sufficient to specifically hybridize under stringent conditions to BMP9 and/ genomic DNA.

In one embodiment, the mRNA is immobilized on a solid surface and contacted with a probe, for example by running the isolated mRNA on an agarose gel and transferring the mRNA from the gel to a membrane, such as nitrocellulose. In an alternative embodiment, the probe(s) are immobilized on a solid surface and the mRNA is contacted with the probe(s), for example, in an Affymetrix gene chip array. A skilled artisan can readily adapt known mRNA detection methods for use in detecting the level of BMP9 and/ mRNA.

An alternative method for determining the level of BMP9 and/ mRNA in a sample involves the process of nucleic acid amplification, *e.g.,* by RT-PCR (the experimental embodiment set forth in Mullis, 1987, U.S. Pat. No. 4,683,202), ligase chain reaction (Barany (1991) Proc. Natl. Acad. Sci. USA 88:189-193), self sustained sequence replication (Guatelli et al. (1990) Proc. Natl. Acad. Sci. USA 87:1874-1878), transcriptional amplification system (Kwoh et al. (1989) Proc. Natl. Acad. Sci. USA 86:1173-1177), Q-Beta Replicase (Lizardi et al. (1988) Bio/Technology 6:1197), rolling circle replication (Lizardi et al., U.S. Pat. No. 5,854,033) or any other nucleic acid amplification method, followed by the detection of the amplified molecules using techniques well known to those of skill in the art. These detection schemes are especially useful for the detection of nucleic acid molecules if such molecules are present in very low numbers. In particular aspects of the invention, BMP9 and/or BMP10expression is assessed by quantitative fluorogenic RT-PCR (*i.e.,* the TaqMan™ System). Such methods typically utilize pairs of oligonucleotide primers that are specific for BMP9 and/or BMP10. Methods for designing oligonucleotide primers specific for a known sequence are well known in the art.

The expression levels of BMP9 mRNA may be monitored using a membrane blot (such as used in hybridization analysis such as Northern, Southern, dot, and the like), or microwells, sample tubes, gels, beads or fibers (or any solid support comprising bound nucleic acids). See U.S. Pat. Nos. 5,770,722, 5,874,219, 5,744,305, 5,677,195 and 5,445,934. The detection of BMP9 expression may also comprise using nucleic acid probes in solution.

In one embodiment of the invention, microarrays are used to detect BMP9 and/ expression. Microarrays are particularly well suited for this purpose because of the reproducibility between different experiments. DNA microarrays provide one method for the simultaneous measurement of the expression levels of large numbers of genes. Each array consists of a reproducible pattern of capture probes attached to a solid support. Labeled RNA or DNA is hybridized to complementary probes on the array and then detected by laser scanning. Hybridization intensities for each probe on the array are determined and converted to a quantitative value representing relative gene expression levels. See, U.S. Pat. Nos. 6,040,138, 5,800,992 and 6,020,135, 6,033,860, and 6,344,316. High-density oligonucleotide arrays are particularly useful for determining the gene expression profile for a large number of RNA's in a sample.

Furthermore, *in vivo* techniques for detection of BMP9 include introducing into a subject a labeled antibody directed against BMP9 and/or BMP10, which binds to and transforms BMP9 into a detectable molecule. As discussed above, the presence, level, or even location of the detectable BMP9 and/ in a subject may be detected determined by standard imaging techniques.

In another embodiment, mass spectrometry can be used to detect BMP9 in a sample. Mass spectrometry is an analytical technique that consists of ionizing chemical compounds to generate charged molecules (or fragments therof) and measuring their mass-to-charge ratios. In a typical mass spectrometry procedure, a sample is obtained from a subject, loaded onto the mass spectrometry, and its components (*e.g*., BMP9 and/ are ionized by different methods (*e.g.,* by impacting them with an electron beam), resulting in the formation of charged particles (ions). The mass-to-charge ratio of the particles is then calculated from the motion of the ions as they transit through electromagnetic fields.

### IV. BMP9 and Antagonism Antibodies

### A. Antibodies

In one embodiment of the invention, the therapeutic and diagnostic methods described herein employ an antibody that binds, *e.g.,* directly to or indirectly to, and inhibits BMP9 activity.

### i. General

The term "antibody" or "immunoglobulin," as used interchangeably herein, includes whole antibodies and any antigen binding fragment (*i.e.,* "antigen-binding portion") or single chains thereof. An "antibody" comprises at least two heavy (H) chains and two light (L) chains inter-connected by disulfide bonds. Each heavy chain is comprised of a heavy chain variable region (abbreviated herein as V_{H}) and a heavy chain constant region. The heavy chain constant region is comprised of three domains, CH1, CH2 and CH3. Each light chain is comprised of a light chain variable region (abbreviated herein as V_{L}) and a light chain constant region. The light chain constant region is comprised of one domain, CL. The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (*e.g.,* effector cells) and the first component (Clq) of the classical complement system.

The term "antigen-binding portion" of an antibody (or simply "antibody portion"), as used herein, refers to one or more fragments of an antibody that retain the ability to specifically bind to an antigen (*e.g.,* BMP9 and/or BMP 10). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the V_{L}, V_{H}, CL and CH1 domains; (ii) a F(ab')₂ fragment, a bivalent fragment comprising two Fab fragments linked by a disulfide bridge at the hinge region; (iii) a Fd fragment consisting of the V_{H} and CH1 domains; (iv) a Fv fragment consisting of the V_{L} and V_{H} domains of a single arm of an antibody, (v) a dAb including VH and VL domains; (vi) a dAb fragment (Ward et al. (1989) Nature 341, 544-546), which consists of a V_{H} domain; (vii) a dAb which consists of a VH or a VL domain; and (viii) an isolated complementarity determining region (CDR) or (ix) a combination of two or more isolated CDRs which may optionally be joined by a synthetic linker. Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); see *e.g.,* Bird et al. (1988) Science 242, 423-426; and Huston et al. (1988) Proc. Natl. Acad. Sci. USA 85, 5879-5883). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies. Antigen-binding portions can be produced by recombinant DNA techniques, or by enzymatic or chemical cleavage of intact immunoglobulins.

The term "monoclonal antibody" as used herein refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. Monoclonal antibodies can be prepared using any art recognized technique and those described herein such as, for example, a hybridoma method, as described by Kohler et al. (1975) Nature, 256:495, a transgenic animal, as described by, for example, (see *e.g.,* Lonberg, et al. (1994) Nature 368(6474): 856-859), recombinant DNA methods (see, *e.g.,* U.S. Pat. No. 4,816,567), or using phage antibody libraries using the techniques described in, for example, Clackson et al., Nature, 352:624-628 (1991) and Marks et al., J. Mol. Biol., 222:581-597 (1991). Monoclonal antibodies include chimeric antibodies, human antibodies and humanized antibodies and may occur naturally or be recombinantly produced.

The term "recombinant antibody," refers to antibodies that are prepared, expressed, created or isolated by recombinant means, such as (a) antibodies isolated from an animal *(e.g.,* a mouse) that is transgenic or transchromosomal for immunoglobulin genes (*e.g.,* human immunoglobulin genes) or a hybridoma prepared therefrom, (b) antibodies isolated from a host cell transformed to express the antibody, *e.g.,* from a transfectoma, (c) antibodies isolated from a recombinant, combinatorial antibody library (*e.g.,* containing human antibody sequences) using phage display, and (d) antibodies prepared, expressed, created or isolated by any other means that involve splicing of immunoglobulin gene sequences (*e.g.,* human immunoglobulin genes) to other DNA sequences. Such recombinant antibodies may have variable and constant regions derived from human germline immunoglobulin sequences. In certain embodiments, however, such recombinant human antibodies can be subjected to *in vitro* mutagenesis and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

The term "chimeric immunoglobulin" or antibody refers to an immunoglobulin or antibody whose variable regions derive from a first species and whose constant regions derive from a second species. Chimeric immunoglobulins or antibodies can be constructed, for example by genetic engineering, from immunoglobulin gene segments belonging to different species.

The term "human antibody," as used herein, is intended to include antibodies having variable regions in which both the framework and CDR regions are derived from human germline immunoglobulin sequences as described, for example, by Kabat et al. (See Kabat, et al. (1991) Sequences of proteins of Immunological Interest, Fifth Edition, U.S. Department of Health and Human Services, NIH Publication No. 91-3242). Furthermore, if the antibody contains a constant region, the constant region also is derived from human germline immunoglobulin sequences. The human antibodies may include amino acid residues not encoded by human germline immunoglobulin sequences (*e.g.,* mutations introduced by random or site-specific mutagenesis *in vitro* or by somatic mutation *in vivo*). However, the term "human antibody", as used herein, is not intended to include antibodies in which CDR sequences derived from the germline of another mammalian species, such as a mouse, have been grafted onto human framework sequences.

The human antibody can have at least one or more amino acids replaced with an amino acid residue, *e.g.,* an activity enhancing amino acid residue which is not encoded by the human germline immunoglobulin sequence. Typically, the human antibody can have up to twenty positions replaced with amino acid residues which are not part of the human germline immunoglobulin sequence. In a particular embodiment, these replacements are within the CDR regions as described in detail below.

The term "humanized immunoglobulin" or "humanized antibody" refers to an immunoglobulin or antibody that includes at least one humanized immunoglobulin or antibody chain (*i. e.,* at least one humanized light or heavy chain). The term "humanized immunoglobulin chain" or "humanized antibody chain" *(i.e.,* a "humanized immunoglobulin light chain" or "humanized immunoglobulin heavy chain") refers to an immunoglobulin or antibody chain (*i.e.,* a light or heavy chain, respectively) having a variable region that includes a variable framework region substantially from a human immunoglobulin or antibody and complementarity determining regions (CDRs) *(e.g.,* at least one CDR, preferably two CDRs, more preferably three CDRs) substantially from a non-human immunoglobulin or antibody, and further includes constant regions *(e.g.,* at least one constant region or portion thereof, in the case of a light chain, and preferably three constant regions in the case of a heavy chain). The term "humanized variable region" (*e.g.,* "humanized light chain variable region" or "humanized heavy chain variable region") refers to a variable region that includes a variable framework region substantially from a human immunoglobulin or antibody and complementarity determining regions (CDRs) substantially from a non-human immunoglobulin or antibody.

A "bispecific" or "bifunctional antibody" is an artificial hybrid antibody having two different heavy/light chain pairs and two different binding sites. Bispecific antibodies can be produced by a variety of methods including fusion of hybridomas or linking of Fab' fragments. See, *e.g.,* Songsivilai & Lachmann, (1990) Clin. Exp. Immunol. 79, 315-321; Kostelny et al. (1992) J. Immunol. 148, 1547-1553.

As used herein, a "heterologous antibody" is defined in relation to the transgenic non-human organism or plant producing such an antibody.

An "isolated antibody," as used herein, is intended to refer to an antibody which is substantially free of other antibodies having different antigenic specificities (*e.g.,* an isolated antibody that specifically binds to BMP9 is substantially free of antibodies that specifically bind antigens other than BMP9). In addition, an isolated antibody is typically substantially free of other cellular material and/or chemicals. In one embodiment of the invention, a combination of "isolated" monoclonal antibodies having different binding specificities are combined in a well defined composition.

As used herein, "isotype" refers to the antibody class *(e.g.,* IgM or IgG1) that is encoded by heavy chain constant region genes. In one embodiment, an antibody or antigen binding portion thereof is of an isotype selected from an IgG1, an IgG2, an IgG3, an IgG4, an IgM, an IgA1, an IgA2, an IgAsec, an IgD, or an IgE antibody isotype.

As used herein, "isotype switching" refers to the phenomenon by which the class, or isotype, of an antibody changes from one Ig class to one of the other Ig classes.

As used herein, "nonswitched isotype" refers to the isotypic class of heavy chain that is produced when no isotype switching has taken place; the CH gene encoding the nonswitched isotype is typically the first CH gene immediately downstream from the functionally rearranged VDJ gene. Isotype switching has been classified as classical or non-classical isotype switching. Classical isotype switching occurs by recombination events which involve at least one switch sequence regions in a gene encoding an antibody. Non-classical isotype switching may occur by, for example, homologous recombination between human σ_{µ} and human Σ_{µ} (δ-associated deletion). Alternative non-classical switching mechanisms, such as intertransgene and/or interchromosomal recombination, among others, may occur and effectuate isotype switching.

As used herein, the term "switch sequence" refers to those DNA sequences responsible for switch recombination. A "switch donor" sequence, typically a µ switch region, will be 5' *(i.e.,* upstream) of the construct region to be deleted during the switch recombination. The "switch acceptor" region will be between the construct region to be deleted and the replacement constant region (*e.g.,* γ, ε, etc.). As there is no specific site where recombination always occurs, the final gene sequence will typically not be predictable from the construct.

The term "epitope" or "antigenic determinant" refers to a site on an antigen to which an immunoglobulin or antibody specifically binds. Epitopes can be formed both from contiguous amino acids or noncontiguous amino acids juxtaposed by tertiary folding of a protein. Epitopes formed from contiguous amino acids are typically retained on exposure to denaturing solvents, whereas epitopes formed by tertiary folding are typically lost on treatment with denaturing solvents. An epitope typically includes at least 3, 4, 5, 6, 7, 8, 9, 10, 11, 12, 13, 14 or 15 amino acids in a unique spatial conformation. Methods of determining spatial conformation of epitopes include techniques in the art and those described herein, for example, x-ray crystallography and 2-dimensional nuclear magnetic resonance. See, *e.g.,* Epitope Mapping Protocols in Methods in Molecular Biology, Vol. 66, G. E. Morris, Ed. (1996).

Antibody proteins obtained from members of the camel and dromedary (*Camelus bactrianus* and *Calelus dromaderius*) family, including New World members such as llama species (*Lama paccos, Lama glama and Lama vicugna)*, have been characterized with respect to size, structural complexity and antigenicity for human subjects. Certain IgG antibodies found in nature in this family of mammals lack light chains, and are thus structurally distinct from the four chain quaternary structure having two heavy and two light chains typical for antibodies from other animals. See, for example, PCT Publication WO 94/04678.

A region of the camelid antibody that is the small, single variable domain identified as V_{HH} can be obtained by genetic engineering to yield a small protein having high affinity for a target, resulting in a low molecular weight, antibody-derived protein known as a "camelid nanobody". See U.S. Pat. No. 5,759,808; see also Stijlemans et al., 2004 J. Biol. Chem. 279: 1256-1261; Dumoulin et al., 2003 Nature 424: 783-788; Pleschberger et al., 2003 Bioconjugate Chem. 14: 440-448; Cortez-Retamozo et al., 2002 Int. J. Cancer 89: 456-62; and Lauwereys. et al., 1998 EMBO J. 17: 3512-3520. Engineered libraries of camelid antibodies and antibody fragments are commercially available, for example, from Ablynx, Ghent, Belgium. As with other antibodies of non-human origin, an amino acid sequence of a camelid antibody can be altered recombinantly to obtain a sequence that more closely resembles a human sequence, *i.e.,* the nanobody can be "humanized". Thus the natural low antigenicity of camelid antibodies to humans can be further reduced.

The camelid nanobody has a molecular weight approximately one-tenth that of a human IgG molecule, and the protein has a physical diameter of only a few nanometers. One consequence of the small size is the ability of camelid nanobodies to bind to antigenic sites that are functionally invisible to larger antibody proteins, i.e., camelid nanobodies are useful as reagents to detect antigens that are otherwise cryptic using classical immunological techniques, and as possible therapeutic agents. Thus, yet another consequence of small size is that a camelid nanobody can inhibit as a result of binding to a specific site in a groove or narrow cleft of a target protein, and hence can serve in a capacity that more closely resembles the function of a classical low molecular weight drug than that of a classical antibody.

The low molecular weight and compact size further result in camelid nanobodies' being extremely thermostable, stable to extreme pH and to proteolytic digestion, and poorly antigenic. Another consequence is that camelid nanobodies readily move from the circulatory system into tissues, and even cross the blood-brain barrier and can treat disorders that affect nervous tissue. Nanobodies can further facilitate drug transport across the blood brain barrier. See U.S. Pat. Pub. No. 20040161738, published August 19, 2004. These features combined with the low antigenicity in humans indicate great therapeutic potential. Further, these molecules can be fully expressed in prokaryotic cells such as *E. coli.*

Accordingly, a feature of the present invention is a camelid antibody or camelid nanobody having high affinity for BMP9 or BMP10. In certain embodiments herein, the camelid antibody or nanobody is naturally produced in the camelid animal, *i.e.,* is produced by the camelid following immunization with BMP9 or a peptide fragment thereof, using techniques described herein for other antibodies. Alternatively, the camelid nanobody is engineered, *i.e.,* produced by selection, for example from a library of phage displaying appropriately mutagenized camelid nanobody proteins using panning procedures. Engineered nanobodies can further be customized by genetic engineering to increase the half life in a recipient subject from 45 minutes to two weeks.

Diabodies are bivalent, bispecific molecules in which V_{H} and V_{L} domains are expressed on a single polypeptide chain, connected by a linker that is too short to allow for pairing between the two domains on the same chain. The V_{H} and V_{L} domains pair with complementary domains of another chain, thereby creating two antigen binding sites (see e.g., Holliger et al., 1993 Proc. Natl. Acad. Sci. USA 90:6444-6448; Poljak et al., 1994 Structure 2:1121-1123). Diabodies can be produced by expressing two polypeptide chains with either the structure V_{HA}-V_{LB} and V_{HB}-V_{LA} (V_{H}-V_{L} configuration), or V_{LA}-V_{HB} and V_{LB}-V_{HA} (V_{L}-V_{H} configuration) within the same cell. Most of them can be expressed in soluble form in bacteria.

Single chain diabodies (scDb) are produced by connecting the two diabody-forming polypeptide chains with linker of approximately 15 amino acid residues (see Holliger and Winter, 1997 Cancer Immunol. Immunother., 45(3-4):128-30; Wu et al., 1996 Immunotechnology, 2(1):21-36). scDb can be expressed in bacteria in soluble, active monomeric form (see Holliger and Winter, 1997 Cancer Immunol. Immunother., 45(34): 128-30; Wu et al., 1996 Immunotechnology, 2(1):21-36; Pluckthun and Pack, 1997 Immunotechnology, 3(2): 83-105; Ridgway et al., 1996 Protein Eng., 9(7):617-21).

A diabody can be fused to Fc to generate a "di-diabody" (see Lu et al., 2004 J. Biol. Chem., 279(4):2856-65).

Antibodies that can be used in the methods of the present invention also include those antibodies that bind the same or an overlapping epitope as the particular antibodies described herein, *i.e*., antibodies that compete for binding to BMP9, or bind to an epitope on BMP9 recognized by the particular antibodies described herein.

Antibodies that recognize the same or an overlapping epitope can be identified using routine techniques such as an immunoassay, for example, by showing the ability of one antibody to block the binding of another antibody to a target antigen, *i.e.,* a competitive binding assay. Competitive binding is determined in an assay in which the immunoglobulin under test inhibits specific binding of a reference antibody to an antigen, such as BMP9 or BMP10. Numerous types of competitive binding assays are known, for example: solid phase direct or indirect radioimmunoassay (RIA), solid phase direct or indirect enzyme immunoassay (EIA), sandwich competition assay (see Stahli et al., (1983) Methods in Enzymology 9:242); solid phase direct biotin-avidin EIA (see Kirkland et al., (1986) J. Immunol. 137:3614); solid phase direct labeled assay, solid phase direct labeled sandwich assay (see Harlow and Lane, (1988) Antibodies: A Laboratory Manual, Cold Spring Harbor Press); solid phase direct label RIA using I-125 label (see Morel et al., (1988) Mol. Immunol. 25(1):7); solid phase direct biotin-avidin EIA (Cheung et al., (1990) Virology 176:546); and direct labeled RIA. (Moldenhauer et al., (1990) Scand. J. Immunol. 32:77). Typically, such an assay involves the use of purified antigen (*e.g.,* BMP9 bound to a solid surface or cells bearing either of these, an unlabeled test immunoglobulin and a labeled reference immunoglobulin. Competitive inhibition is measured by determining the amount of label bound to the solid surface or cells in the presence of the test immunoglobulin. Usually the test immunoglobulin is present in excess. Usually, when a competing antibody is present in excess, it will inhibit specific binding of a reference antibody to a common antigen by at least 50-55%, 55-60%, 60-65%, 65-70% 70-75% or more.

As used herein, the terms "specific binding," "specifically binds," "selective binding," and "selectively binds," mean that an antibody or antigen-binding portion thereof, exhibits appreciable affinity for a particular antigen or epitope and, generally, does not exhibit significant cross-reactivity with other antigens and epitopes. "Appreciable" or preferred binding includes binding with an affinity of at least 10⁶, 10⁷, 10⁸, 10⁹ M⁻¹, or 10¹⁰ M⁻¹. Affinities greater than 10⁷ M⁻¹, preferably greater than 10⁸ M⁻¹ are more preferred. Values intermediate of those set forth herein are also intended to be within the scope of the present invention and a preferred binding affinity can be indicated as a range of affinities, for example, 10⁶ to 10¹⁰ M⁻¹, preferably 10⁷ to 10¹⁰ M⁻¹, more preferably 10⁸ to 10¹⁰ M⁻¹. An antibody that "does not exhibit significant cross-reactivity" is one that will not appreciably bind to an undesirable entity (*e.g.,* an undesirable proteinaceous entity). Specific or selective binding can be determined according to any art-recognized means for determining such binding, including, for example, according to Scatchard analysis and/or competitive binding assays.

The term "K_{D}," as used herein, is intended to refer to the dissociation equilibrium constant of a particular antibody-antigen interaction or the affinity of an antibody for an antigen. In one embodiment, the antibody or antigen binding portion thereof according to the present invention binds an antigen (*e.g.,* BMP9 with an affinity (K_{D}) of 50 nM or better (*i. e.,* or less) (*e.g.,* 40 nM or 30 nM or 20 nM or 10 nM or less), as measured using a surface plasmon resonance assay or a cell binding assay. In a particular embodiment, an antibody or antigen binding portion thereof according to the present invention binds BMP9 with an affinity (K_{D}) of 8 nM or better (*e.g.,* 7 nM, 6 nM, 5 nM, 4 nM, 2 nM, 1.5 nM, 1.4 nM, 1.3 nM, 1nM or less), as measured by a surface plasmon resonance assay or a cell binding assay. In other embodiments, an antibody or antigen binding portion thereof binds an antigen (*e.g*., BMP9 with an affinity (K_{D}) of approximately less than 10⁻⁷ M, such as approximately less than 10⁻⁸ M, 10⁻⁹ M or 10⁻¹⁰ M or even lower when determined by surface plasmon resonance (SPR) technology in a BIACORE 3000 instrument using recombinant BMP9 as the analyte and the antibody as the ligand, and binds to the predetermined antigen with an affinity that is at least two-fold greater than its affinity for binding to a non-specific antigen (*e.g.,* BSA, casein) other than the predetermined antigen or a closely-related antigen.

The term "K_{off}", as used herein, is intended to refer to the off rate constant for the dissociation of an antibody from the antibody/antigen complex.

The term "EC50," as used herein, refers to the concentration of an antibody or an antigen-binding portion thereof, which induces a response, either in an *in vitro* or an *in vivo* assay, which is 50% of the maximal response, *i.e.,* halfway between the maximal response and the baseline.

As used herein, "glycosylation pattern" is defined as the pattern of carbohydrate units that are covalently attached to a protein, more specifically to an immunoglobulin protein.

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory is naturally-occurring.

The term "rearranged" as used herein refers to a configuration of a heavy chain or light chain immunoglobulin locus wherein a V segment is positioned immediately adjacent to a D-J or J segment in a conformation encoding essentially a complete V_{H} or V_{L} domain, respectively. A rearranged immunoglobulin gene locus can be identified by comparison to germline DNA; a rearranged locus will have at least one recombined heptamer/nonamer homology element.

The term "unrearranged" or "germline configuration" as used herein in reference to a V segment refers to the configuration wherein the V segment is not recombined so as to be immediately adjacent to a D or J segment.

The term "modifying," or "modification," as used herein, is intended to refer to changing one or more amino acids in the antibodies. The change can be produced by adding, substituting or deleting an amino acid at one or more positions. The change can be produced using known techniques, such as PCR mutagenesis. For example, in some embodiments, an antibody employed by the methods of the present invention can be modified, to thereby modify the binding affinity of the antibody to BMP9 or BMP10.

The present invention also encompasses "conservative amino acid substitutions" in the sequences of the antibodies used in the methods of the invention, *i.e.,* nucleotide and amino acid sequence modifications which do not abrogate the binding of the antibody encoded by the nucleotide sequence or containing the amino acid sequence, to the antigen, *i.e.,* BMP9 or BMP10. Conservative amino acid substitutions include the substitution of an amino acid in one class by an amino acid of the same class, where a class is defined by common physicochemical amino acid side chain properties and high substitution frequencies in homologous proteins found in nature, as determined, for example, by a standard Dayhoff frequency exchange matrix or BLOSUM matrix. Six general classes of amino acid side chains have been categorized and include: Class I (Cys); Class II (Ser, Thr, Pro, Ala, Gly); Class III (Asn, Asp, Gln, Glu); Class IV (His, Arg, Lys); Class V (Ile, Leu, Val, Met); and Class VI (Phe, Tyr, Trp). For example, substitution of an Asp for another class III residue such as Asn, Gln, or Glu, is a conservative substitution. Thus, a predicted nonessential amino acid residue in an anti-BMP9 antibody of the present invention is preferably replaced with another amino acid residue from the same class. Methods of identifying nucleotide and amino acid conservative substitutions which do not eliminate antigen binding are well-known in the art (see, *e.g.,* Brummell et al., Biochem. 32:1180-1187 (1993); Kobayashi et al. Protein Eng. 12(10):879-884 (1999); and Burks et al. Proc. Natl. Acad. Sci. USA 94:.412-417 (1997)).

The term "non-conservative amino acid substitution" refers to the substitution of an amino acid in one class with an amino acid from another class; for example, substitution of an Ala, a class II residue, with a class III residue such as Asp, Asn, Glu, or Gln.

Alternatively, in another embodiment, mutations (conservative or non-conservative) can be introduced randomly along all or part of an anti-BMP9 antibody coding sequence, such as by saturation mutagenesis, and the resulting modified anti-BMP9 antibodies can be screened for binding activity.

A "consensus sequence" is a sequence formed from the most frequently occurring amino acids (or nucleotides) in a family of related sequences (See *e.g.,* Winnaker, From Genes to Clones (Verlagsgesellschaft, Weinheim, Germany 1987). In a family of proteins, each position in the consensus sequence is occupied by the amino acid occurring most frequently at that position in the family. If two amino acids occur equally frequently, either can be included in the consensus sequence. A "consensus framework" of an immunoglobulin refers to a framework region in the consensus immunoglobulin sequence.

### ii. Engineered and modified antibodies

An antibody of the invention can be prepared using an antibody having one or more V_{H} and/or V_{L} sequences as starting material to engineer a modified antibody, which modified antibody may have altered properties from the starting antibody. An antibody can be engineered by modifying one or more residues within one or both variable regions (*i.e.,* V_{H} and/or V_{L}), for example within one or more CDR regions and/or within one or more framework regions. Additionally or alternatively, an antibody can be engineered by modifying residues within the constant region(s), for example to alter the effector function(s) of the antibody.

One type of variable region engineering that can be performed is CDR grafting. Antibodies interact with target antigens predominantly through amino acid residues that are located in the six heavy and light chain CDRs. For this reason, the amino acid sequences within CDRs are more diverse between individual antibodies than sequences outside of CDRs. Because CDR sequences are responsible for most antibody-antigen interactions, it is possible to express recombinant antibodies that mimic the properties of specific naturally occurring antibodies by constructing expression vectors that include CDR sequences from the specific naturally occurring antibody grafted onto framework sequences from a different antibody with different properties (see, e.g., Riechmann et al., 1998 Nature 332:323-327; Jones et al., 1986 Nature 321:522-525; Queen et al., 1989 Proc. Natl. Acad. See. U.S.A. 86:10029-10033; U.S. Pat. No. 5,225,539, and U.S. Pat. Nos. 5,530,101; 5,585,089; 5,693,762 and 6,180,370).

Framework sequences can be obtained from public DNA databases or published references that include germline antibody gene sequences. For example, germline DNA sequences for human heavy and light chain variable region genes can be found in the "VBase" human germline sequence database (available on the Internet at www.mrc-cpe.cam.ac.uk/vbase), as well as in Kabat et al., 1991 Sequences of Proteins of Immunological Interest, Fifth Edition, U.S. Department of Health and Human Services, NIH Publication No. 91-3242; Tomlinson et al., 1992 J. Mol. Biol. 227:776-798; and Cox et al., 1994 Eur. J. Immunol. 24:827-836.

The V_{H} CDR1, 2 and 3 sequences and the V_{L} CDR1, 2 and 3 sequences can be grafted onto framework regions that have the identical sequence as that found in the germline immunoglobulin gene from which the framework sequence is derived, or the CDR sequences can be grafted onto framework regions that contain one or more mutations as compared to the germline sequences. For example, it has been found that in certain instances it is beneficial to mutate residues within the framework regions to maintain or enhance the antigen binding ability of the antibody (see e.g., U.S. Pat. Nos. 5,530,101; 5,585,089; 5,693,762 and 6,180,370).

CDRs can also be grafted into framework regions of polypeptides other than immunoglobulin domains. Appropriate scaffolds form a conformationally stable framework that displays the grafted residues such that they form a localized surface and bind the target of interest (*e.g.,* BMP9 or BMP 10). For example, CDRs can be grafted onto a scaffold in which the framework regions are based on fibronectin, ankyrin, lipocalin, neocarzinostain, cytochrome b, CP1 zinc finger, PST1, coiled coil, LACI-D1, Z domain or tendramisat (See e.g., Nygren and Uhlen, 1997 Current Opinion in Structural Biology, 7, 463-469).

Another type of variable region modification is mutation of amino acid residues within the V_{H} and/or V_{L} CDR1, CDR2 and/or CDR3 regions to thereby improve one or more binding properties (e.g., affinity) of the antibody of interest, known as "affinity maturation." Site-directed mutagenesis or PCR-mediated mutagenesis can be performed to introduce the mutation(s), and the effect on antibody binding, or other functional property of interest, can be evaluated in in vitro or in vivo assays as described herein. Conservative modifications can be introduced. The mutations may be amino acid substitutions, additions or deletions. Moreover, typically no more than one, two, three, four or five residues within a CDR region are altered.

Engineered antibodies of the invention include those in which modifications have been made to framework residues within V_{H} and/or V_{L}, e.g., to improve the properties of the antibody. Typically such framework modifications are made to decrease the immunogenicity of the antibody. For example, one approach is to "backmutate" one or more framework residues to the corresponding germline sequence. More specifically, an antibody that has undergone somatic mutation may contain framework residues that differ from the germline sequence from which the antibody is derived. Such residues can be identified by comparing the antibody framework sequences to the germline sequences from which the antibody is derived. To return the framework region sequences to their germline configuration, the somatic mutations can be "backmutated" to the germline sequence by, for example, site-directed mutagenesis or PCR-mediated mutagenesis. Such "backmutated" antibodies are also intended to be encompassed by the invention.

Another type of framework modification involves mutating one or more residues within the framework region, or even within one or more CDR regions, to remove T cell - epitopes to thereby reduce the potential immunogenicity of the antibody. This approach is also referred to as "deimmunization" and is described in further detail in U.S. Pat. Pub. No. 20030153043 by Carr et al.

In addition or alternative to modifications made within the framework or CDR regions, antibodies of the invention may be engineered to include modifications within the Fc region, typically to alter one or more functional properties of the antibody, such as serum half-life, complement fixation, Fc receptor binding, and/or antigen-dependent cellular cytotoxicity. Furthermore, an antibody of the invention may be chemically modified (e.g., one or more chemical moieties can be attached to the antibody) or be modified to alter its glycosylation, again to alter one or more functional properties of the antibody.

In one embodiment, the hinge region of CH1 is modified such that the number of cysteine residues in the hinge region is altered, e.g., increased or decreased. This approach is described further in U.S. Pat. No. 5,677,425 by Bodmer et al*.* The number of cysteine residues in the hinge region of CH1 is altered to, for example, facilitate assembly of the light and heavy chains or to increase or decrease the stability of the antibody.

In another embodiment, the Fc hinge region of an antibody is mutated to decrease the biological half-life of the antibody. More specifically, one or more amino acid mutations are introduced into the CH2-CH3 domain interface region of the Fc-hinge fragment such that the antibody has impaired Staphylococcyl protein A (SpA) binding relative to native Fc-hinge domain SpA binding. This approach is described in further detail in U.S. Pat. No. 6,165,745 by Ward et al*.*

In another embodiment, the antibody is modified to increase its biological half-life. Various approaches are possible. For example, U.S. Pat. No. 6,277,375 describes the following mutations in an IgG that increase its half-life in vivo: T252L, T254S, T256F. Alternatively, to increase the biological half life, the antibody can be altered within the CH1 or CL region to contain a salvage receptor binding epitope taken from two loops of a CH2 domain of an Fc region of an IgG, as described in U.S. Pat. Nos. 5,869,046 and 6,121,022 by Presta et al.

In yet other embodiments, the Fc region is altered by replacing at least one amino acid residue with a different amino acid residue to alter the effector functions of the antibody. For example, one or more amino acids can be replaced with a different amino acid residue such that the antibody has an altered affinity for an effector ligand but retains the antigen-binding ability of the parent antibody. The effector ligand to which affinity is altered can be, for example, an Fc receptor or the C1 component of complement. This approach is described in further detail in U.S. Pat. Nos. 5,624,821 and 5,648,260, both by Winter et al.

In another embodiment, one or more amino acids selected from amino acid residues can be replaced with a different amino acid residue such that the antibody has altered C1q binding and/or reduced or abolished complement dependent cytotoxicity (CDC). This approach is described in further detail in U.S. Pat. Nos. 6,194,551 by Idusogie et al.

In another embodiment, one or more amino acid residues are altered to thereby alter the ability of the antibody to fix complement. This approach is described further in WO 94/29351 by Bodmer et al.

In yet another embodiment, the Fc region is modified to increase the ability of the antibody to mediate antibody dependent cellular cytotoxicity (ADCC) and/or to increase the affinity of the antibody for an Fcγ receptor by modifying one or more amino acids. This approach is described further in WO 00/42072 by Presta. Moreover, the binding sites on human IgG1 for FcγR1, FcγRII, FcγRIII and FcRn have been mapped and variants with improved binding have been described (see Shields, R.L. et al., 2001 J. Biol. Chem. 276:6591-6604).

In still another embodiment, the glycosylation of an antibody is modified. For example, an aglycoslated antibody can be made (*i.e.,* the antibody lacks glycosylation). Glycosylation can be altered, for example, to increase the affinity of the antibody for an antigen. Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. Such aglycosylation may increase the affinity of the antibody for antigen. Such an approach is described in further detail in U.S. Pat. Nos. 5,714,350 and 6,350,861 by Co et al.

Additionally or alternatively, an antibody can be made that has an altered type of glycosylation, such as a hypofucosylated antibody having reduced amounts of fucosyl residues or an antibody having increased bisecting GlcNac structures. Such altered glycosylation patterns have been demonstrated to increase the ADCC ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies of the invention to thereby produce an antibody with altered glycosylation. For example, EP 1,176,195 by Hang et al. describes a cell line with a functionally disrupted FUT8 gene, which encodes a fucosyl transferase, such that antibodies expressed in such a cell line exhibit hypofucosylation. PCT Pub. WO 03/035835 by Presta describes a variant CHO cell line, Lecl3 cells, with reduced ability to attach fucose to Asn(297)-linked carbohydrates, also resulting in hypofucosylation of antibodies expressed in that host cell (see also Shields, R.L. et al., 2002 J. Biol. Chem. 277:26733-26740). WO 99/54342 by Umana et al. describes cell lines engineered to express glycoprotein-modifying glycosyl transferases (*e.g.,* beta(1,4)-N acetylglucosaminyltransferase III (GnTIII)) such that antibodies expressed in the engineered cell lines exhibit increased bisecting GlcNac structures which results in increased ADCC activity of the antibodies (see also Umana et al., 1999 Nat. Biotech. 17:176-180).

Another modification of the antibodies herein that is contemplated by the invention is pegylation. An antibody can be pegylated to, for example, increase the biological (*e.g.,* serum) half-life of the antibody. To pegylate an antibody, the antibody, or fragment thereof, typically is reacted with polyethylene glycol (PEG), such as a reactive ester or aldehyde derivative of PEG, under conditions in which one or more PEG moieties become attached to the antibody or antibody fragment. The pegylation can be carried out by an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono (C1-C10) alkoxy- or aryloxy-polyethylene glycol or polyethylene glycol-maleimide. In certain embodiments, the antibody to be pegylated is an aglycosylated antibody. Methods for pegylating proteins are known in the art and can be applied to the antibodies of the invention. See for example, EP 0 154 316 by Nishimura et al. and EP 0 401 384 by Ishikawa et al.

In addition, pegylation can be achieved in any part of a BMP9 binding polypeptide of the invention by the introduction of a nonnatural amino acid. Certain nonnatural amino acids can be introduced by the technology described in Deiters et al., J Am Chem Soc 125:11782-11783, 2003; Wang and Schultz, Science 301:964-967, 2003; Wang et al., Science 292:498-500, 2001; Zhang et al., Science 303:371-373, 2004 or in US Patent No. 7,083,970. Briefly, some of these expression systems involve site-directed mutagenesis to introduce a nonsense codon, such as an amber TAG, into the open reading frame encoding a polypeptide of the invention. Such expression vectors are then introduced into a host that can utilize a tRNA specific for the introduced nonsense codon and charged with the nonnatural amino acid of choice. Particular nonnatural amino acids that are beneficial for purpose of conjugating moieties to the polypeptides of the invention include those with acetylene and azido side chains. The polypeptides containing these novel amino acids can then be pegylated at these chosen sites in the protein.

### iii. Antibody Fragments

The instant invention is not limited to traditional antibodies and may be practiced through the use of antibody fragments. As detailed below, a wide variety of antibody fragment technologies have now been developed and are widely known in the art.

Domain Antibodies (dAbs) are the smallest functional binding units of antibodies, corresponding to the variable regions of either the heavy (VH) or light (VL) chains of human antibodies. Domain Antibodies have a molecular weight of approximately 13 kDa. Domantis has developed a series of large and highly functional libraries of fully human VH and VL dAbs (more than ten billion different sequences in each library), and uses these libraries to select dAbs that are specific to therapeutic targets. In contrast to many conventional antibodies, domain antibodies are well expressed in bacterial, yeast, and mammalian cell systems. Further details of domain antibodies and methods of production thereof may be obtained by reference to U.S. Patent 6,291,158; 6,582,915; 6,593,081; 6,172,197; 6,696,245; U.S. Serial No. 2004/0110941; European patent application No. 1433846 and European Patents 0368684 & 0616640; WO05/035572, WO04/10179 WO04/081026, WO04/058821, WO04/003019 and WO03/002609. Nanobodies are antibody-derived therapeutic proteins that contain the unique structural and functional properties of naturally-occurring heavy-chain antibodies. These heavy-chain antibodies contain a single variable domain (VHH) and two constant domains (CH2 and CH3). Importantly, the cloned and isolated VHH domain is a perfectly stable polypeptide harbouring the full antigen-binding capacity of the original heavy-chain antibody. Nanobodies have a high homology with the VH domains of human antibodies and can be further humanized without any loss of activity. Importantly, Nanobodies have a low immunogenic potential, which has been confirmed in primate studies with Nanobody lead compounds.

Nanobodies combine the advantages of conventional antibodies with important features of small molecule drugs. Like conventional antibodies, Nanobodies show high target specificity, high affinity for their target and low inherent toxicity. However, like small molecule drugs they can inhibit enzymes and readily access receptor clefts. Furthermore, Nanobodies are extremely stable, can be administered by means other than injection (see, *e.g.,* WO 04/041867) and are easy to manufacture. Other advantages of Nanobodies include recognizing uncommon or hidden epitopes as a result of their small size, binding into cavities or active sites of protein targets with high affinity and selectivity due to their unique 3-dimensional, drug format flexibility, tailoring of half-life and ease and speed of drug discovery.

Nanobodies are encoded by single genes and are efficiently produced in almost all prokaryotic and eukaryotic hosts, *e.g.,* E. coli (see, *e.g.,* U.S. 6,765,087, which is herein incorporated by reference in its entirety), molds (for example Aspergillus or Trichoderma) and yeast (for example Saccharomyces, Kluyveromyces, Hansenula or Pichia) (see, *e.g.,* U.S. 6,838,254). The production process is scalable and multi-kilogram quantities of Nanobodies have been produced. Because Nanobodies exhibit a superior stability compared with conventional antibodies, they can be formulated as a long shelf-life, ready-to-use solution.

The Nanoclone method (see, *e.g.,* WO 06/079372) is a proprietary method for generating Nanobodies against a desired target, based on automated high-throughout selection of B-cells and could be used in the context of the instant invention.

UniBodies are another antibody fragment technology, however this one is based upon the removal of the hinge region of IgG4 antibodies. The deletion of the hinge region results in a molecule that is essentially half the size of traditional IgG4 antibodies and has a univalent binding region rather than the bivalent binding region of IgG4 antibodies. It is also well known that IgG4 antibodies are inert and thus do not interact with the immune system, which may be advantageous for the treatment of diseases where an immune response is not desired, and this advantage is passed onto UniBodies. For example, UniBodies may function to inhibit or silence, but not kill, the cells to which they are bound. Additionally, UniBody binding to cancer cells do not stimulate them to proliferate. Furthermore, because UniBodies are about half the size of traditional IgG4 antibodies, they may show better distribution over larger solid tumors with potentially advantageous efficacy. UniBodies are cleared from the body at a similar rate to whole IgG4 antibodies and are able to bind with a similar affinity for their antigens as whole antibodies. Further details of UniBodies may be obtained by reference to patent application WO2007/059782.

### B. Immunoconjugates

In another aspect, the methods of present invention employ immunoconjugate agents that target BMP9 and which inhibit or down-modulate BMP9. Agents that can be targeted to BMP9 include, but are not limited to, cytotoxic agents, anti-inflammatory agents, *e.g.,* a steroidal or nonsteroidal inflammatory agent, or a cytotoxin antimetabolites (*e.g.,* methotrexate, 6-mercaptopurine, 6-thioguanine, cytarabine, 5-fluorouracil decarbazine), alkylating agents (*e.g.,* mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (*e.g.,* daunorubicin (formerly daunomycin) and doxorubicin), antibiotics (*e.g.,* dactinomycin (formerly actinomycin), bleomycin, mithramycin, and anthramycin (AMC)), and anti-mitotic agents (*e.g.,* vincristine and vinblastine).

The term "cytotoxin" or "cytotoxic agent" includes any agent that is detrimental *(e.g.,* kills) to fibrotic tissue. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof.

Immunoconjugates can be formed by conjugating (*e.g.,* chemically linking or recombinantly expressing) antibodies to suitable therapeutic agents. Suitable agents include, for example, a cytotoxic agent, a toxin (*e.g.* an enzymatically active toxin of bacterial, fungal, plant or animal origin, or fragments thereof), and/or a radioactive isotope (*i.e.,* a radioconjugate). Enzymatically active toxins and fragments thereof which can be used include diphtheria A chain, nonbinding active fragments of diphtheria toxin, exotoxin A chain (from *Pseudomonas aeruginosa*), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. A variety of radionuclides are available for the production of radioconjugated antibodies. Examples include ²¹² Bi, ¹³¹ I, ¹³¹ In, ⁹⁰Y and ¹⁸⁶Re.

Immunoconjugates can be made using a variety of bifunctional protein coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody (see, *e.g.,* WO94/11026).

### EXAMPLES

### Example 1

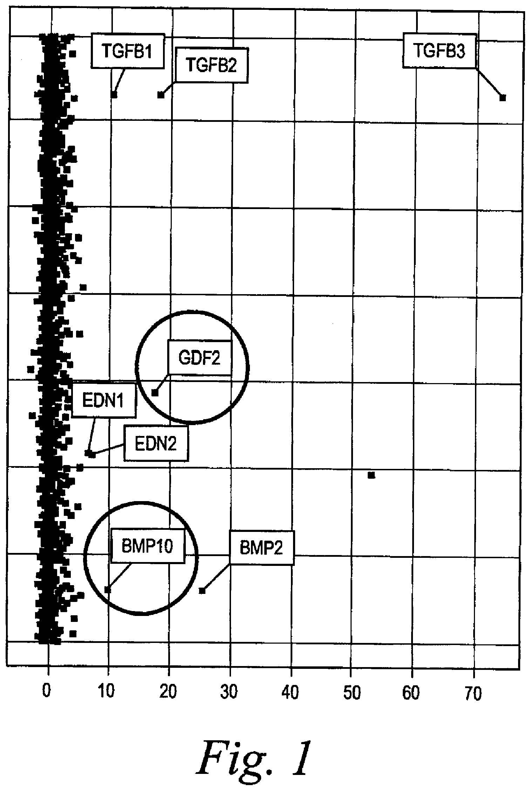

BMP9 and BMP10 were identified as indicators of fibrosis in a phenotypic screen and, therefore, as potential therapeutic targets capable of treating fibrotic disorders. The experiment was performed in 384 well plate format and in triplicate. Briefly, a reverse transfection in Hek293 cells of approximately 1500 human cDNA expression clones encoding predicted secreted proteins was performed using Fugene 6 (Roche) at a ratio of 4:1 (Fugene:DNA) in complete DMEM (Invitrogen) medium. Hek293 cells were washed 24 hours post transfection and incubated for an additional 48 hours to enrich for secreted proteins. Conditioned media from the transfected Hek293 cells were transferred onto human primary dermal fibroblast at passage 3 (Lonza) that were synchronized by starvation for 24 hours in absence of serum. The differentiation of fibroblast into myofibroblast was scored 96 hours later by immunostaining for the presence of alpha smooth muscle actin (αSMA [Sigma], a known marker of fibroblast differentiation) using high content imaging (Figure 1). Three fields of view were imaged using an In Cell Analyzer (GE Healthcare) and pixel intensity per nuclei was calculated as a measure of percent differentiation.

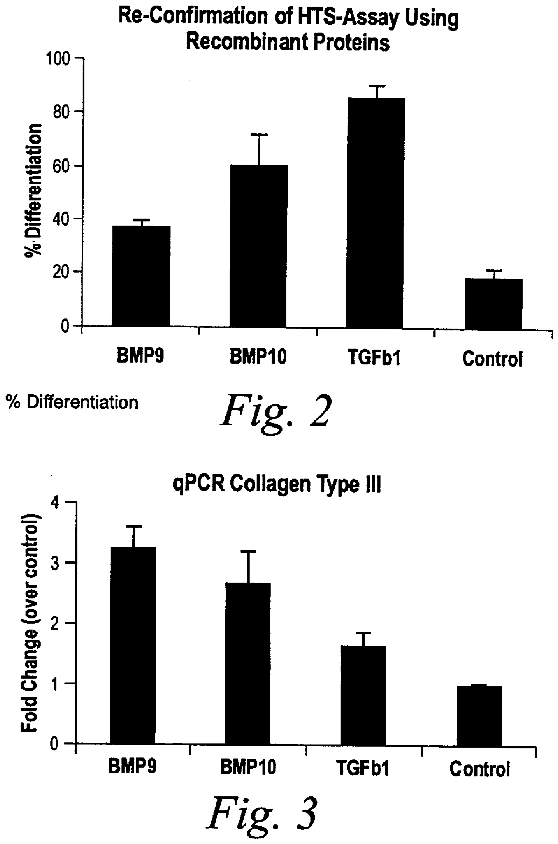

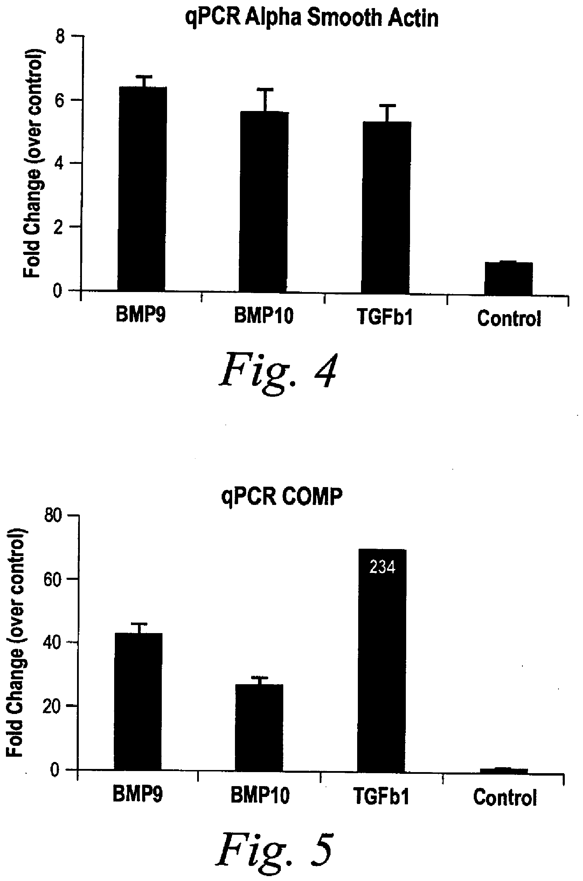

To confirm the activity of BMP9 and BMP10 on fibroblast differentiation, recombinant BMP9 and BMP 10 protein was obtained (R&D) and used to stimulate resting fibroblast (Figure 2). Briefly, 40ng/ml of active BMP9 recombinant protein and 1000ng/ml of active BMP10 recombinant protein (R&D System) were serially diluted 2 fold and used in a 12 dose response experiment using human primary dermal fibroblast at passage 3 that were synchronized by starvation for 24 hours in absence of serum. The differentiation of fibroblast into myofibroblast was scored 96 hours later by immunostaining for the presence of alpha smooth muscle actin using high content imaging. BMP9 and BMP 10 were shown to positively modulate differentiation of fibroblasts at an effective concentration (EC50) of approximately 2 ng/ml and approximately 125 ng/ml, respectively. In addition, treatment of fibroblasts with recombinant BMP9 or BMP10 proteins used at an EC90 dose of 10ng/ml and 250ng/ml respectively for 24-96 hours also led to the up-regulation of known markers of fibrosis. Briefly, human primary dermal fibroblast at passage 3 that were synchronized by starvation for 24 hours in absence of serum then treated with BMP9, BMP10 or with 10ng/ml TGFb1. Fibroblasts were collected at various time post stimulation (24-96 hours) and RNAs were prepared (Qiagen) for use in qPCR assays. Modulation of known fibrotic markers including alpha smooth muscle actin, collagen type III and cartilage oligomeric matrix protein (COMP) by TGFb, BMP9 or BMP10 was determined (Figures 3-5). Both BMP9 and BMP10 were shown to induce transcriptional activity of alpha smooth muscle actin, collagen type III and cartilage oligomeric matrix protein.

### Example 2

To further assess the role of BMP9 in fibrosis, a reporter gene assay (RGA) was established. Specifically, a BMP9 reporter construct (ID-BRE) was generated using Id1 promoter BMP-response element (BRE) fused with firefly luciferase gene. The BMP9 RGA was conducted on HEK293 cells stably transfected with ID-BRE. HEK293 ID-BRE cells were seeded at 5x10⁵ cells/35 mm-dish one day before transfection. 2.5 µg DNA constructs of constitutively active ALK1 and GFP (10:1) were co-transfected into HEK293 ID-BRE cells via Fugene 6 (Roche, 8µl), followed by manufacturer's protocol on day 2. Transfection efficiency was measured by visualization of GFP. Transfected cells were then seeded into 96-well plate at 1x10⁴ cells/well on day 3. BMP9 was added to the 96-well plate at different concentration with or without a BMP9 neutralizing antibody or a soluble receptor, ALK1-Fc, on day 4. Cells were harvested on day 5 and luciferase activity was measured using Bright-Glo Luciferase Assay System (Promega) following manufacturer's protocol.

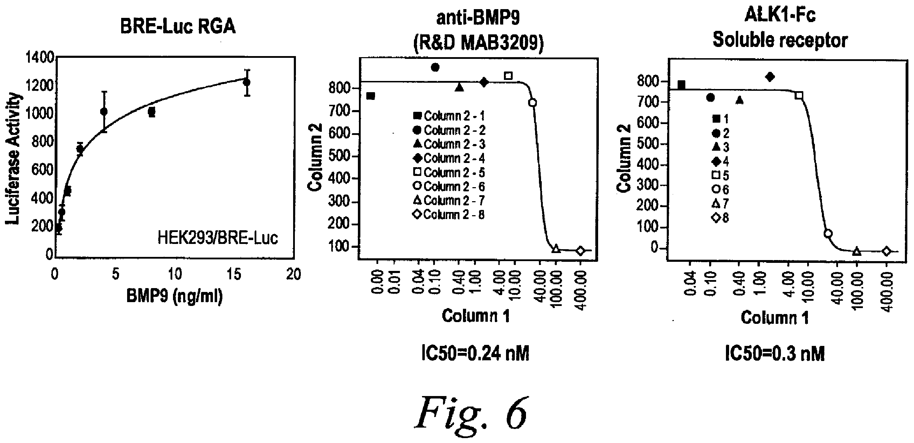

As shown in Figure 6, both an anti-BMP9 neutralizing antibody and a soluble receptor, ALK1-Fc, inhibited BMP9-stimulated BRE-luciferase activity.

### Example 3

An Epithelial-Mesenchymal Transdifferentiation (EMT) assay was performed to assess whether BMP9 has any effect on EMT in hepatocytes. Specifically, Hep3B cells (ATCC) were cultured in DMEM-F12, supplemented with 10% FBS, 10 U /ml penicillin, and 10 U /ml streptomycin, at 37°C in a humidified atmosphere containing 5% CO2. 2 x 10⁵ Hep3B cells were seeded in complete DMEM-F12 into each well of 24-well plates. The medium was replaced with low serum (1%) medium with or without 10 ng/ml of BMP9 (R&D).

In one experiment, the cells were fixed 24 hours post-treatment with or without BMP9, fixed for image analysis and subjected to immunohistochemistry staining with E-cadherin and α-SMA. As evidenced by the cell morphological changes depicted in Figure 6, BMP9 induced EMT, decreased E-cadherin and increased α-SMA, as compared to the control.

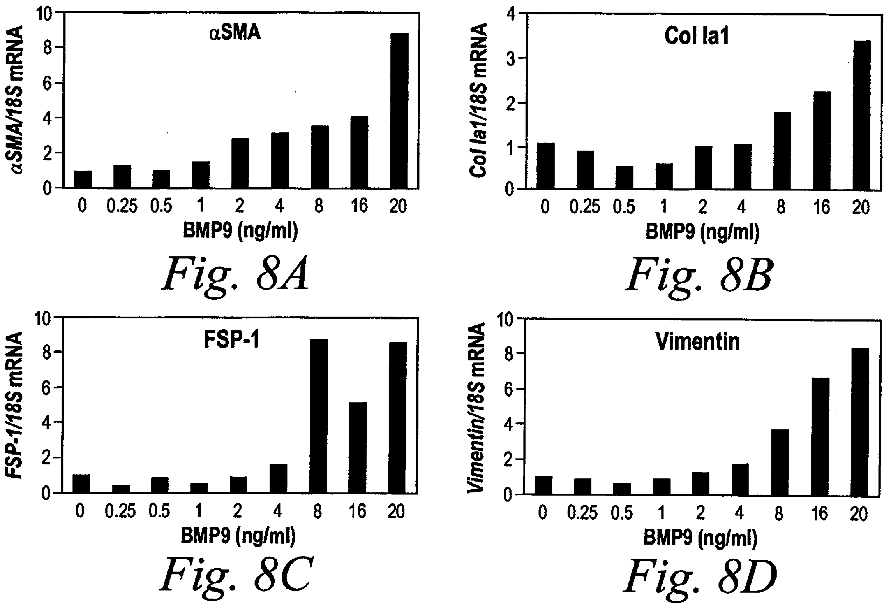

For quantitative PCR analysis, the cells were harvested 48 hours post-BMP9 treatment and the total RNA was extracted and subjected to RT-PCR analysis. The PCR was run in ABI7700HT. As shown in Figures 8A-8D, BMP9 induced expression of four different fibroblast markers (*i.e.,* αSMA, Col la1, fibroblast specific protein (FSP-1), and vimentin) in a dose-dependent manner.

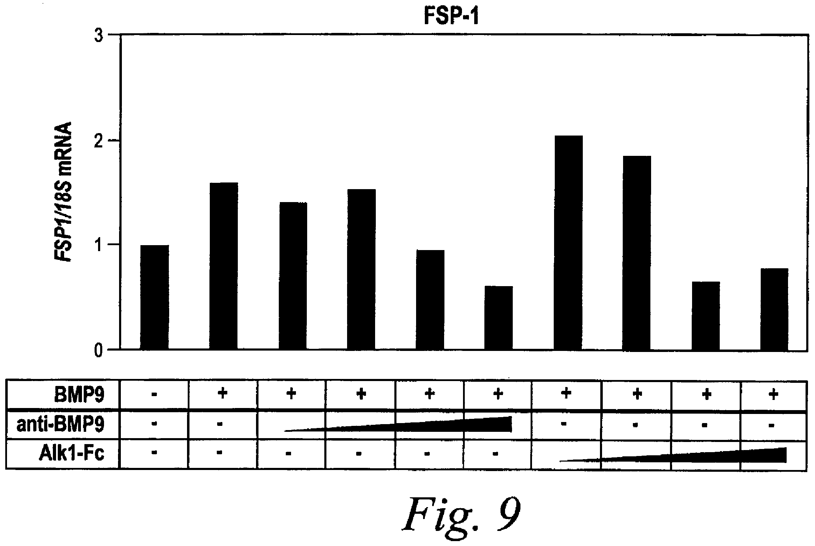

The cells were also treated with BMP9 in the absence or presence of an anti-BMP9 antibody or ALK1-Fc. 48 hours post-treatment, the total fibroblast specific protein (FSP-1) RNA was extracted and subjected for qPCR analysis. As shown in Figure 9, both a BMP9 neutralizing monoclonal antibody and a soluble receptor (ALK1-Fc) inhibited BMP9-induced FSP-1 expression.

### Equivalents

Those skilled in the art will recognize, or be able to ascertain using no more than routine experimentation, many equivalents of the specific embodiments of the invention described herein.

## Claims

1. An anti-BMP9 antagonist antibody for use in a method of treating or preventing a fibrotic disorder in a subject, particularly a human, comprising administering to said subject an effective amount of an anti-BMP9 antibody.

2. The anti-BMP9 antagonist antibody for use according to claim 1, wherein the fibrotic disorder is selected from the group consisting of vascular fibrosis, pulmonary fibrosis, pancreatic fibrosis, liver fibrosis, renal fibrosis, musculoskeletal fibrosis, cardiac fibrosis, skin fibrosis, eye fibrosis, glaucoma, progressive systemic sclerosis (PSS), chronic graft versus-host disease, scleroderma, Peyronie's disease, post-cystoscopic urethral stenosis, idiopathic and pharmacologically induced retroperitoneal fibrosis, mediastinal fibrosis, progressive massive fibrosis, proliferative fibrosis and neoplastic fibrosis.

3. The anti-BMP9 antagonist antibody for use according to any one of claims 1-2, wherein said antibody is administered to said subject
a) together with an additional therapeutic agent; and/or
b) intravenously, intramuscularly, or subcutaneously.

4. The anti-BMP9 antagonist antibody for use according to any one of claims 1-3, wherein said antibody is selected from the group consisting of
a) a murine antibody, a human antibody, a humanized antibody, a bispecific antibody and a chimeric antibody, or
b) a Fab, Fab'2, ScFv, SMIP, affibody, and a domain antibody.

5. The anti-BMP9 antagonist antibody for use according to any one of claims 1-4, wherein the fibrotic disorder is selected from the group consisting of liver fibrosis, kidney fibrosis, heart fibrosis, skin fibrosis, and lung fibrosis.

6. The anti-BMP9 antagonist antibody for use in a method of treating or preventing a fibrotic disorder according to claim 6 wherein the fibrotic disorder is liver fibrosis.

7. A method for assessing whether a subject has or is at risk of developing a fibrotic disorder comprising: (i) contacting a sample from said subject with a reagent able to detect BMP9; and (ii) detecting BMP9, wherein an elevated level of BMP9 relative to a control is an indication that the subject has or is at risk of developing a fibrotic disorder.

8. The method of claim 7 further comprising detecting an additional fibrosis marker selected from the group consisting of alpha smooth muscle actin, collagen type III cartilage oligomeric matrix protein, collagen type I, collagen type IV, fibroblast specific protein-1, fibronectin, serpinEl, periostin, IGFBP3, SPARC, CTGF, TGFb, Cyr61, BMP10 and phospho smad 2/3.

9. The method of claim 7, wherein the reagent is
a) an antibody or a nucleic acid; and/or
b) is detectably labeled, particularly with a label selected from the group consisting of a radioisotope, a bioluminescent compound, a chemiluminescent compound, a fluorescent compound, a metal chelate, or an enzyme.

10. The method of claim 7, wherein the sample comprises
a) cells obtained from the subject; or
b) a fluid obtained from the subject, particularly a fluid selected from the group consisting of blood fluids, lymph, gynecological fluids, cystic fluid, ocular fluid, urine, and fluids collected by peritoneal rinsing.

11. The method of claim 7, wherein the level of BMP9 is (a) at least 2-fold, (b) at least 3-fold, higher relative to the control.

12. A method of assessing the efficacy of a treatment regimen for treating a fibrotic disorder in a subject, the method comprising:
a) contacting a first sample obtained from said subject prior to administering at least a portion of the treatment regimen to the subject with a reagent able to detect BMP9;
b) contacting a second sample obtained from said subject following administration of at least a portion of the treatment regimen with a reagent able to detect BMP9; and
c) comparing the levels of BMP9 from the first and second samples,
wherein an elevated level of BMP9 present in the first sample, relative to the second sample, is an indication that the treatment regimen is efficacious for treating a fibrotic disorder in the subject.

13. The method of claim 12, wherein the treatment regimen comprises administration of an anti-BMP9 antibody.

14. The method of claim 13, wherein the anti-BMP9 antibody is selected from the group consisting of
a) a murine antibody, a human antibody, a humanized antibody, a bispecific antibody and a chimeric antibody, or
b) a Fab, Fab'2, ScFv, SMIP, affibody and a domain antibody.

15. The method of any one of claims 12-14, wherein the fibrotic disorder is selected from the group consisting of vascular fibrosis, pulmonary fibrosis, pancreatic fibrosis, liver fibrosis, renal fibrosis, musculoskeletal fibrosis, cardiac fibrosis, skin fibrosis, eye fibrosis, glaucoma, progressive systemic sclerosis (PSS), chronic graft versus-host disease, scleroderma, Peyronie's disease, post-cystoscopic urethral stenosis, idiopathic and pharmacologically induced retroperitoneal fibrosis, mediastinal fibrosis, progressive massive fibrosis, proliferative fibrosis and neoplastic fibrosis.

## Patentansprüche

1. Anti-BMP9-Antagonisten-Antikörper zur Verwendung bei einem Verfahren zur Behandlung oder Prävention einer fibrotischen Störung in einem Individuum, insbesondere einem Menschen, umfassend das Verabreichen einer wirksamen Menge eines Anti-BMP9-Antikörpers an das Individuum.

2. Anti-BMP9-Antagonisten-Antikörper zur Verwendung nach Anspruch 1, wobei die fibrotische Störung ausgewählt ist aus der Gruppe bestehend aus Gefäßfibrose, Lungenfibrose, Pankreasfibrose, Leberfibrose, Nierenfibrose, Muskel-Skelett-Fibrose, Herzfibrose, Hautfibrose, Augenfibrose, Glaukom, progressiver System-Sklerose (PSS), chronischer Transplantat-versus-Wirts-Erkrankung, Sklerodermie, Peyronie-Krankheit, postzystoskopischer Harnröhrenstenose, idiopathischer und pharmakologisch bedingter retroperitonealer Fibrose, mediastinaler Fibrose, progressiver massiver Fibrose, proliferativer Fibrose und neoplastischer Fibrose.

3. Anti-BMP9-Antagonisten-Antikörper zur Verwendung nach einem der Ansprüche 1 bis 2, wobei der Antikörper dem Individuum
a) zusammen mit einem zusätzlichen Therapeutikum; und/oder
b) intravenös, intramuskulär oder subkutan verabreicht wird.

4. Anti-BMP9-Antagonisten-Antikörper zur Verwendung nach einem der Ansprüche 1 bis 3, wobei der Antikörper ausgewählt ist aus der Gruppe, bestehend aus
a) einem Maus-Antikörper, einem Human-Antikörper, einem humanisierten Antikörper, einem bispezifischen Antikörper und einem chimären Antikörper, oder
b) einem Fab, Fab'2, ScFv, SMIP, Affibody und einem Domänen-Antikörper.

5. Anti-BMP9-Antagonisten-Antikörper zur Verwendung nach einem der Ansprüche 1 bis 4, wobei die fibrotische Störung ausgewählt ist aus der Gruppe bestehend aus Leberfibrose, Nierenfibrose, Herzfibrose, Hautfibrose und Lungenfibrose.

6. Anti-BMP9-Antagonisten-Antikörper zur Verwendung bei einem Verfahren zur Behandlung oder Prävention einer fibrotischen Störung nach Anspruch 6, wobei die fibrotische Störung Leberfibrose ist.

7. Verfahren zur Beurteilung, ob ein Individuum eine fibrotische Störung aufweist oder gefährdet ist, eine solche zu entwickeln, umfassend: (i) Inkontaktbringen einer Probe aus dem Individuum mit einem Reagenz, das BMP9 erfassen kann; und (ii) Erfassen von BMP9, wobei ein erhöhter Spiegel von BMP9 relativ zu einer Kontrolle ein Hinweis darauf ist, dass das Individuum eine fibrotische Störung aufweist oder gefährdet ist, eine solche zu entwickeln.

8. Verfahren nach Anspruch 7, zudem umfassend das Nachweisen eines zusätzlichen Fibrose-Markers, ausgewählt aus der Gruppe bestehend aus Alpha-Aktin aus glattem Muskel, Kollagen-Typ-III-Knorpel-Oligomer-Matrix-Protein, Kollagen Typ I, Kollagen Typ IV, Fibroblasten-spezifischem Protein-1, Fibronectin, SerpinEl, Periostin, IGFBP3, SPARC, CTGF, TGFb, Cyr61, BMP10 und Phospho smad 2/3.

9. Verfahren nach Anspruch 7, wobei das Reagenz
a) ein Antikörper oder eine Nukleinsäure ist; und/oder
b) nachweisbar markiert ist, insbesondere mit einer Markierung ausgewählt aus der Gruppe bestehend aus einem Radioisotop, einer biolumineszierenden Verbindung, einer chemilumineszierenden Verbindung, einer fluoreszierenden Verbindung, einem Metallchelat oder einem Enzym.

10. Verfahren nach Anspruch 7, wobei die Probe
a) aus dem Individuum erhaltene Zellen; oder
b) eine Flüssigkeit, die aus dem Individuum erhalten wird, insbesondere eine Flüssigkeit ausgewählt aus der Gruppe, bestehend aus Blutflüssigkeiten, Lymphe, gynäkologischen Flüssigkeiten, zystischer Flüssigkeit, Augenflüssigkeit, Urin und Flüssigkeiten, die durch Peritonealspülung gesammelt werden,
umfasst.

11. Verfahren nach Anspruch 7, wobei der Spiegel von BMP9 gegenüber der Kontrolle (a) mindestens zweifach, (b) mindestens dreifach höher ist.

12. Verfahren zur Beurteilung der Wirksamkeit eines Behandlungsschemas zur Behandlung einer fibrotischen Störung bei einem Individuum, wobei das Verfahren umfasst:
a) Inkontaktbringen einer ersten Probe, die aus dem Individuum vor der Verabreichung mindestens eines Teils des Behandlungsschemas an das Individuum erhalten wurde, mit einem Reagenz, das BMP9 erfassen kann;
b) Inkontaktbringen einer zweiten Probe, die aus dem Individuum nach Verabreichung von mindestens einem Teil des Behandlungsschemas erhalten wurde, mit einem Reagenz, das BMP9 erfassen kann; und
c) Vergleichen der Spiegel von BMP9 aus der ersten und der zweiten Probe, wobei ein erhöhter Spiegel von BMP9, der in der ersten Probe vorhanden ist, relativ zu der zweiten Probe, ein Hinweis darauf ist, dass das Behandlungsschema für die Behandlung einer fibrotischen Störung in dem Individuum wirksam ist.

13. Verfahren nach Anspruch 12, wobei das Behandlungsschema die Verabreichung eines Anti-BMP9-Antikörpers umfasst.

14. Verfahren nach Anspruch 13, wobei der Anti-BMP9-Antikörper ausgewählt ist aus der Gruppe, bestehend aus
a) einem Maus-Antikörper, einem Human-Antikörper, einem humanisierten Antikörper, einem bispezifischen Antikörper und einem chimären Antikörper, oder
b) einem Fab, Fab'2, ScFv, SMIP, Affibody und einem Domänen-Antikörper.

15. Verfahren nach einem der Ansprüche 12 bis 14, wobei die fibrotische Störung ausgewählt ist aus der Gruppe, bestehend aus Gefäßfibrose, Lungenfibrose, Pankreasfibrose, Leberfibrose, Nierenfibrose, Muskel-Skelett-Fibrose, Herzfibrose, Hautfibrose, Augenfibrose, Glaukom, progressiver System-Sklerose (PSS), chronischer Transplantat-versus-Wirts-Erkrankung, Sklerodermie, Peyronie-Krankheit, postzystoskopischer Harnröhrenstenose, idiopathischer und pharmakologisch bedingter retroperitonealer Fibrose, mediastinaler Fibrose, progressiver massiver Fibrose, proliferativer Fibrose und neoplastischer Fibrose.

## Revendications

1. Anticorps antagoniste anti-BMP9 destiné à être utilisé dans une méthode de traitement ou de prévention d'un trouble fibreux chez un sujet, en particulier un être humain, comprenant l'administration audit sujet d'une quantité efficace d'un anticorps anti-BMP9.

2. Anticorps antagoniste anti-BMP9 destiné à être utilisé selon la revendication 1, le trouble fibreux étant choisi dans le groupe constitué par la fibrose vasculaire, la fibrose pulmonaire, la fibrose pancréatique, la fibrose du foie, la fibrose rénale, la fibrose musculo-squelettique, la fibrose cardiaque, la fibrose de la peau, la fibrose de l'oeil, le glaucome, la sclérodermie systémique progressive (PSS), la maladie chronique du greffon contre l'hôte, la sclérodermie, la maladie de La Peyronie, la sténose urétrale post-cytoscopie, la fibrose rétropéritonéale idiopathique et d'origine pharmacologique, la fibrose médiastinale, la fibrose massive progressive, une fibrose proliférative et une fibrose néoplasique.

3. Anticorps antagoniste anti-BMP9 destiné à être utilisé selon l'une quelconque des revendications 1-2, ledit anticorps étant administré audit sujet
a) conjointement avec un agent thérapeutique supplémentaire ; et/ou
b) par voie intraveineuse, par voie intramusculaire ou par voie sous-cutanée.

4. Anticorps antagoniste anti-BMP9 destiné à être utilisé selon l'une quelconque des revendications 1-3, ledit anticorps étant choisi dans le groupe constitué par
a) un anticorps murin, un anticorps humain, un anticorps humanisé, un anticorps bispécifique et un anticorps chimérique ou
b) un Fab, Fab'2, ScFv, SMIP, afficorps et un anticorps à domaine unique.

5. Anticorps antagoniste anti-BMP9 destiné à être utilisé selon l'une quelconque des revendications 1-4, le trouble fibreux étant choisi dans le groupe constitué par la fibrose du foie, la fibrose du rein, la fibrose du cour, la fibrose de la peau et la fibrose du poumon.

6. Anticorps antagoniste anti-BMP9 destiné à être utilisé dans une méthode de traitement ou de prévention d'un trouble fibreux selon la revendication 6, le trouble fibreux étant la fibrose du foie.

7. Procédé permettant d'évaluer si un sujet a ou présente un risque de développer un trouble fibreux comprenant : (i) la mise en contact d'un échantillon provenant dudit sujet avec un réactif pouvant détecter BMP9 ; et (ii) la détection de BMP9, un taux élevé de BMP9 par rapport à un témoin étant une indication que le sujet a ou présente un risque de développer un trouble fibreux.

8. Procédé selon la revendication 7 comprenant en outre la détection d'un marqueur de fibrose supplémentaire choisi dans le groupe constitué par l'alpha-actine du muscle lisse, le collagène de type III, la protéine oligomère de la matrice du cartilage, le collagène de type I, le collagène de type IV, la protéine-1 spécifique des fibroblastes, la fibronectine, la serpine E1, la périostine, l'IGFBP3, la SPARC, le CTGF, le TGFb, la Cyr61, la BMP10 et le phospho-Smad2/3.

9. Procédé selon la revendication 7, le réactif étant
a) un anticorps ou un acide nucléique ; et/ou
b) marqué de façon détectable, en particulier avec un marqueur choisi dans le groupe constitué par un radioisotope, un composé bioluminescent, un composé chimiluminescent, un composé fluorescent, un chélate métallique ou une enzyme.

10. Procédé selon la revendication 7, l'échantillon comprenant
a) des cellules obtenues à partir du sujet ; ou
b) un liquide obtenu à partir du sujet, en particulier un liquide choisi dans le groupe constitué par les liquides sanguins, la lymphe, les liquides gynécologiques, un liquide kystique, un liquide oculaire, l'urine et les liquides prélevés par rinçage péritonéal.

11. Procédé selon la revendication 7, le taux de BMP9 étant (a) au moins 2 fois, (b) au moins 3 fois plus élevé par rapport au témoin.

12. Procédé d'évaluation de l'efficacité d'un régime de traitement pour le traitement d'un trouble fibreux chez un sujet, le procédé comprenant :
a) la mise en contact d'un premier échantillon obtenu à partir dudit sujet avant l'administration d'au moins une partie du régime de traitement au sujet avec un réactif pouvant détecter BMP9 ;
b) la mise en contact d'un second échantillon obtenu à partir dudit sujet après l'administration d'au moins une partie du régime de traitement avec un réactif pouvant détecter BMP9 ; et
c) la comparaison des taux de BMP9 des premier et second échantillons,
un taux élevé de BMP9 présente dans le premier échantillon, par rapport au second échantillon, étant une indication que le régime de traitement est efficace pour le traitement d'un trouble fibreux chez le sujet.

13. Procédé selon la revendication 12, le régime de traitement comprenant l'administration d'un anticorps anti-BMP9.

14. Procédé selon la revendication 13, l'anticorps anti-BMP9 étant choisi dans le groupe constitué par
a) un anticorps murin, un anticorps humain, un anticorps humanisé, un anticorps bispécifique et un anticorps chimérique ou
b) un Fab, Fab'2, ScFv, SMIP, afficorps et un anticorps à domaine unique.

15. Procédé selon l'une quelconque des revendications 12-14, le trouble fibreux étant choisi dans le groupe constitué par la fibrose vasculaire, la fibrose pulmonaire, la fibrose pancréatique, la fibrose du foie, la fibrose rénale, la fibrose musculo-squelettique, la fibrose cardiaque, la fibrose de la peau, la fibrose de l'oeil, le glaucome, la sclérodermie systémique progressive (PSS), la maladie chronique du greffon contre l'hôte, la sclérodermie, la maladie de La Peyronie, la sténose urétrale post-cytoscopie, la fibrose rétropéritonéale idiopathique et d'origine pharmacologique, la fibrose médiastinale, la fibrose massive progressive, une fibrose proliférative et une fibrose néoplasique.
